# EUROPEAN PATENT SPECIFICATION

(11) **EP 4 484 431 B1**
(45) Date of publication and mention of the grant of the patent: **09.12.2026**
(21) Application number: 24195355.3
(22) Date of filing: 23.06.2020
(51) Int. Cl.: C07D 207/16

(54) **PROCESSES AND INTERMEDIATES FOR PRODUCING DIAZASPIRO LACTAM COMPOUNDS**
VERFAHREN UND ZWISCHENPRODUKTE ZUR HERSTELLUNG VON DIAZASPIROLACTAMVERBINDUNGEN
PROCÉDÉS ET INTERMÉDIAIRES POUR LA PRODUCTION DE COMPOSÉS DE DIAZASPIRO LACTAME

(30) Priority: 24.06.2019 US 201962865840 P
(43) Date of publication of application: 01.01.2025
(62) Document number(s) of the earlier application(s) in accordance with Art. 76 EPC: 20832011.9 / 3 986 400
(73) Proprietor: Naurex Inc., North Chicago, IL 60064 (US)
(72) Inventor: DINH, Danny, T, Costa Mesa, CA 92626 (US); PERRAULT, William R., Kalamazoo, MI 49004 (US); DIKER, Khalid, 60340 Saint Leu D'esserent (FR)
(74) Representative: Hoffmann Eitle

(56) References cited:
- WO-A1-2014/120783
- WO-A1-2014/120786
- WO-A1-2017/201283
- WO-A1-2019/152696
- WO-A1-2020/263847

## Description

### TECHNICAL FIELD

The present invention relates to compounds useful for producing diazaspiro lactam compounds.

### BACKGROUND

N-methyl-D-aspartate receptor (NMDA receptor) is believed to play a major role in the synaptic plasticity that underlies many higher cognitive functions, such as memory acquisition, retention and learning, as well as in certain cognitive pathways and in the perception of pain. The NMDA receptor also appears to be involved in a broad spectrum of CNS disorders. NMDA receptor modulators therefore can provide pharmaceutical benefits.

U.S. 9,512,134 discloses NMDA receptor modulators that can be useful for treating, for example, depression. There remains a need for economical and facile processes for the preparation of the diazaspiro lactam compounds described herein.

WO 2019/152696, which is prior art under Article 54(3) EPC, WO 2014/130786, WO 2017/201283 and WO 2014/120783 disclose spiro-lactam NMDA receptor modulators.

### SUMMARY

In one aspect (aspect 1), a compound of formula II is provided: 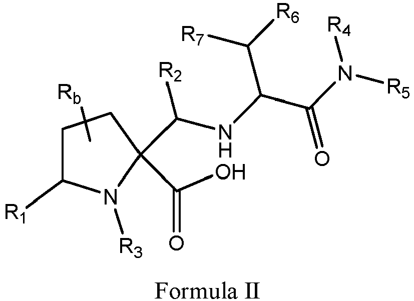 wherein:
R_{b} is selected from H, halogen, cyano and C₁-C₆ alkyl;
R₁ is H or C₁-C₆ alkyl;
R₂ is H or C₁-C₆ alkyl;
R₃ is selected from C₁-C₆ alkyl and C(O)OR₈;
R₈ is selected from C₁-C₆ alkyl, C₁-C₆ haloalkyl, C₂-C₆ alkenyl, C₂-C₆ alkynyl, C₃-C₁₀ cycloalkyl, and -CH₂-C₃-C₁₀ cycloalkyl, wherein C₃-C₁₀ cycloalkyl is optionally substituted with one, two, or three groups selected from C₁-C₃ alkyl;
R₄ and R₅ are independently selected from H, C₁-C₆ alkyl, X, and -C₁-C₆ alkylene-X, wherein X is selected from
   (i) C₃-C₆ cycloalkyl;
   (ii) heteroaryl containing from 5 to 6 ring atoms wherein one, two, or three of the ring atoms are independently selected from N, NH, N(C₁-C₃ alkyl), O, and S;
   (iii) heterocyclyl containing from three to six ring atoms wherein 1, 2, or 3 of the ring atoms are independently selected from N, NH, N(C₁-C₃ alkyl), O, and S; and
   (iv) phenyl;
   wherein C₃-C₆ cycloalkyl, heterocyclyl, heteroaryl, and phenyl are each optionally substituted with one, two, or three substituents independently selected halogen, cyano, C₁-C₆ alkyl, hydroxyl, and C₁-C₆ alkoxy;
or R₄ and R₅ together with the nitrogen to which they are attached form heterocyclyl containing from 4 to 6 ring atoms; wherein the heterocyclyl contains not more than two ring heteroatoms including the nitrogen atom attached to R₄ and R₅, and the second ring heteroatom, when present, is independently selected from N-C₁-C₆ alkyl, O and S; and wherein the heterocyclyl is optionally substituted with one, two, or three substituents independently selected from halogen, cyano, C₁-C₆ alkyl, and C₁-C₆ alkoxy;
R₆ is selected from -OH, C₁-C₆ alkoxy, -OC(O)-C₁-C₆ alkyl, and -OC(O)phenyl; and
R₇ is H or C₁-C₆ alkyl.

This compound is useful in a process of producing a compound of formula I or a pharmaceutically acceptable salt thereof, the process comprises contacting the compound of formula II and a coupling agent in an organic solvent to obtain a compound of formula I or a pharmaceutically acceptable salt thereof:

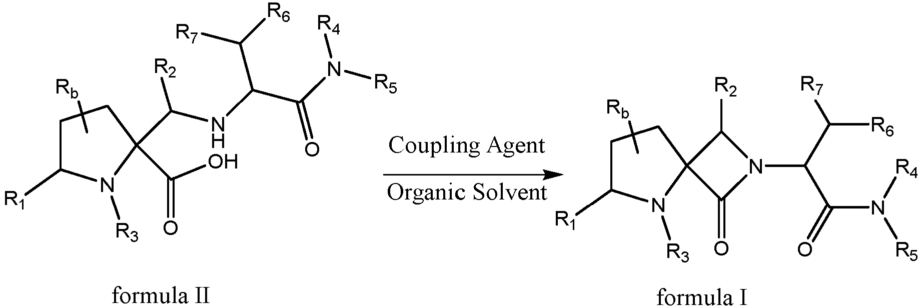

In another aspect (aspect 2), a compound that is useful in the process of producing the diazaspiro lactam compound of formula I is provided. The compound is a compound of formula III: 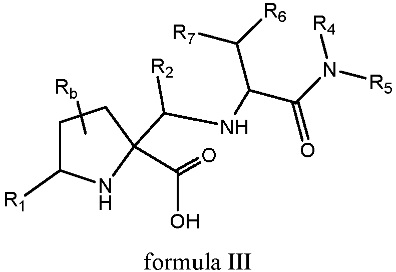 wherein R_{b}, R₁, R₂, R₄, R₅, R₆, and R₇ are the same as described for formula II.

Some non-limiting example embodiments are listed below.

Example embodiment 1: The compound of aspect 1, which is a compound of formula IIa:

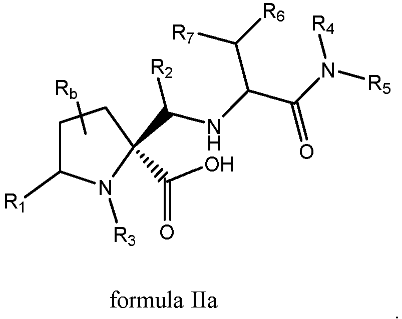

Example embodiment 2: The compound of example embodiment 1, which is a compound of formula IIb:

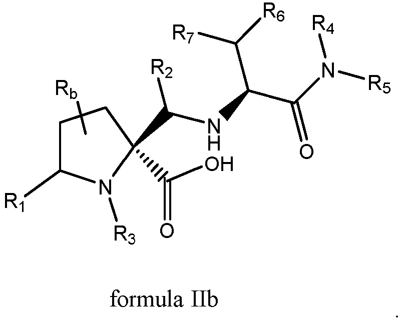

Example embodiment 3: The compound of example embodiment 2, which is a compound of formula IIc:

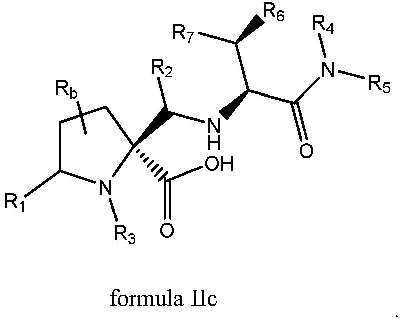

**Example embodiment 4:** The compound of any one of aspect 1 and example embodiments 1 to 3, wherein R₄ and R₅ are independently selected from H and C₁-C₆ alkyl; or R₄ and R₅ together with the nitrogen to which they are attached form heterocyclyl containing from 4 to 6 ring atoms; wherein the heterocyclyl contains not more than two ring heteroatoms (including the nitrogen atom attached to R₄ and R₅), and the second ring heteroatom, when present, is independently selected from N-C₁-C₆ alkyl, O and S; and wherein the heterocyclyl is optionally substituted with one, two, or three substituents independently selected from halogen, cyano, C₁-C₆ alkyl, and C₁-C₆ alkoxy.

**Example embodiment** 5: The compound of example embodiment 4, wherein R₁ and R₂ are H.

**Example embodiment** 6: The compound of example embodiment 4, wherein each of R₁, R₂, R₄, and R₅ is H.

**Example embodiment** 7: The compound of any one of example embodiments 4 to 6, wherein R₆ is OH.

**Example embodiment** 8: The compound of any one of example embodiments 4 to 7, wherein R₇ is C₁-C₆ alkyl.

**Example embodiment** 9: The compound of example embodiment 8, wherein R₇ is methyl.

**Example embodiment** 10: The compound of any one of example embodiments 4 to 9, wherein R₃ is OC(O)R₈ and R₈ is C₁-C₆ alkyl.

**Example embodiment** 11: The compound of example embodiment 10, wherein R₈ is tertiary butyl.

**Example embodiment** 12: The compound of aspect 1, which is selected from the following compounds:

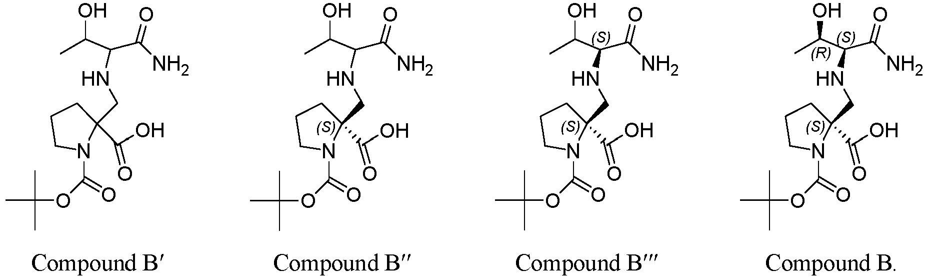

Example embodiment 13: The compound of example embodiment 12, which is Compound B.

Example embodiment 14: The compound of aspect 2, which is a compound of formula IIIa:

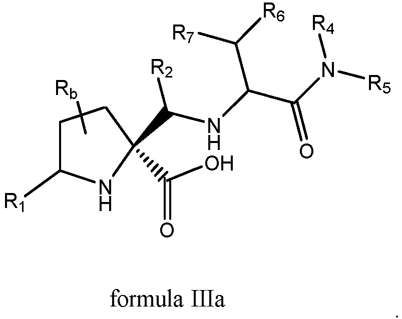

Example embodiment 15: The compound of example embodiment 14, which is a compound of formula IIIb:

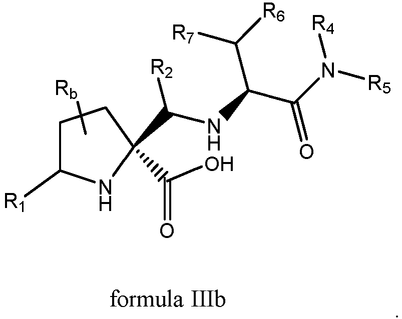

Example embodiment 16: The compound of example embodiment 15, which is a compound of formula IIIc:

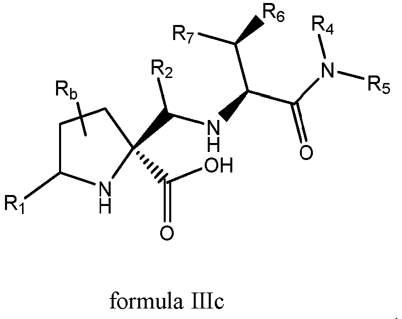

Example embodiment 17: The compound of any one of aspect 2 and example embodiments 14 to 16, wherein R₄ and R₅ are independently selected from H and C₁-C₆ alkyl; or R₄ and R₈ together with the nitrogen to which they are attached form heterocyclyl containing from 4 to 6 ring atoms; wherein the heterocyclyl contains not more than two ring heteroatoms (including the nitrogen atom attached to R₄ and R₅), and the second ring heteroatom, when present, is independently selected from N-C₁-C₆ alkyl, O and S; and wherein the heterocyclyl is optionally substituted with one, two, or three substituents independently selected from halogen, cyano, C₁-C₆ alkyl, and C₁-C₆ alkoxy.

Example embodiment 18: The compound of example embodiment 17, wherein R₁ and R₂ are H.

Example embodiment 19: The compound of example embodiment 17, wherein each of R₁, R₂, R₄, and R₅ is H.

Example embodiment 20: The compound of any one of example embodiments 17 to 19, wherein R₆ is OH.

**Example embodiment** 21: The compound of any one of example embodiments 17 to 20, wherein R₇ is C₁-C₆ alkyl.

**Example embodiment** 22: The compound of example embodiment 21, wherein R₇ is methyl.

**Example embodiment** 23: The compound of any one of example embodiments 17 to 22, wherein R₃ is OC(O)R₈ and R₈ is C₁-C₆ alkyl.

**Example embodiment** 24: The compound of example embodiment 23, wherein R₈ is tertiary butyl.

**Example embodiment** 25: The compound of aspect 2, which is selected from the following compounds:

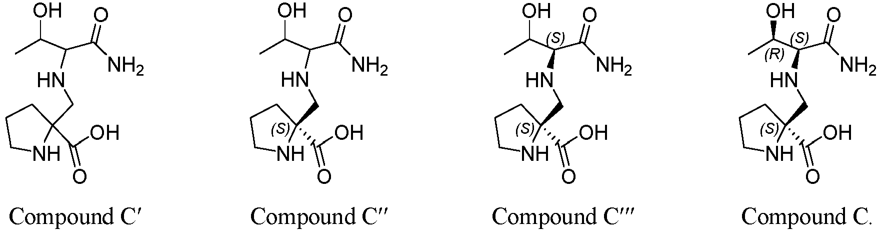

**Example embodiment 26:** The compound of example embodiment 25, which is Compound C.

### DETAILED DESCRIPTION

### I. Definitions

As used herein, the term "alkenyl" refers to an unsaturated straight or branched hydrocarbon having at least one carbon-carbon double bond, such as a straight or branched group of 2-6 or 3-4 carbon atoms, referred to herein for example as C₂-C₆ alkenyl, and C₃-C₄ alkenyl, respectively. Exemplary alkenyl groups include, but are not limited to, vinyl, allyl, butenyl, pentenyl, etc.

The term "alkyl" as used herein refers to a saturated straight or branched hydrocarbon, such as a straight or branched group of 1 to 6, 1 to 4, or 1 to 3 carbon atoms, referred to herein as C₁-C₆ alkyl, C₁-C₄ alkyl, and C₁-C₃ alkyl, respectively. Exemplary alkyl groups include, but are not limited to, methyl, ethyl, propyl, isopropyl, 2-methyl-1-propyl, 2-methyl-2-propyl, 2-methyl-1-butyl, 3-methyl-1-butyl, 3-methyl-2-butyl, 2,2-dimethyl-1-propyl, 2-methyl-1-pentyl, 3-methyl-1-pentyl, 4-methyl-1-pentyl, 2-methyl-2-pentyl, 3-methyl-2-pentyl, 4-methyl-2-pentyl, 2,2-dimethyl-1-butyl, 3,3-dimethyl-1-butyl, 2-ethyl-1-butyl, butyl, isobutyl, t-butyl, pentyl, isopentyl, neopentyl, hexyl, etc.

The term "alkoxy" as used herein refers to a straight or branched alkyl group attached to an oxygen (alkyl-O-). Exemplary alkoxy groups include, but are not limited to, alkoxy groups of 1-6 or 2 to 6 carbon atoms, referred to herein as C₁-C₆ alkoxy, and C₂-C₆ alkoxy, respectively. Exemplary alkoxy groups include, but are not limited to methoxy, ethoxy, isopropoxy, etc.

The term "alkylene" as used herein refers to a bivalent saturated straight or branched hydrocarbon, such as a straight or branched group of 1 to 6, 1 to 4, or 1 to 3 carbon atoms, referred to herein as C₁-C₆ alkylene, C₁-C₄ alkylene, and C₁-C₃ alkylene, respectively. Exemplary alkylene groups include, but are not limited to, methylene (-CH₂-), ethylene (-CH₂CH₂-), propylene (-CH₂CH₂CH₂-), and isopropylene (-CH₂CH(CH₃)).

The term "alkynyl" as used herein refers to an unsaturated straight or branched hydrocarbon having at least one carbon-carbon triple bond, such as a straight or branched group of 2-6, or 3-6 carbon atoms, referred to herein as C₂-C₆ alkynyl, and C₃-C₆ alkynyl, respectively. Exemplary alkynyl groups include, but are not limited to, ethynyl, propynyl, butynyl, pentynyl, hexynyl, methylpropynyl, etc.

The term "cycloalkyl" as used herein refers to a monocyclic saturated or partially unsaturated (non-aromatic) hydrocarbon group of for example 3-6, or 4-6 carbons, referred to herein, e.g., as "C₃-C₆ cycloalkyl" or "C₄-C₆ cycloalkyl," and derived from a cycloalkane. Exemplary cycloalkyl groups include, but are not limited to, cyclohexane, cyclohexene, cyclopentane, cyclobutane, cyclopropane or cyclopentane.

The term "haloalkyl" as used herein refers to a saturated straight or branched alkyl groups, in which one or more hydrogen atoms of the alkyl group are replaced with one or more independently selected halogens. The term "haloalkyl" encompasses alkyl groups in which all of hydrogen atoms of the alkyl group are replaced independently selected halogens (sometimes referred to as "perhalo" alkyl groups. Exemplary haloalkyl groups include, but are not limited to, CH₂F, CH₂CH₂Cl, CF₃, CHFCH₂Cl.

The terms "heteroaryl" as used herein refers to a monocyclic aromatic 4-6 membered ring system containing one or more heteroatoms, for example one to three heteroatoms, such as nitrogen, oxygen, and sulfur. Where possible, said heteroaryl ring may be linked to the adjacent radical though carbon or nitrogen. Examples of heteroaryl rings include but are not limited to furan, thiophene, pyrrole, thiazole, oxazole, isothiazole, isoxazole, imidazole, pyrazole, triazole, pyridine, and pyrimidine.

The terms "heterocyclyl" or "heterocyclic group" as used herein refer to saturated or partially unsaturated (but not aromatic) 4- to 7-membered ring structures, whose ring structures include one to three heteroatoms, such as nitrogen, oxygen, and sulfur. A heterocycle may be fused to one or more phenyl, partially unsaturated, or saturated rings. Examples of heterocyclyl groups include but are not limited to pyrrolidine, piperidine, morpholine, thiomorpholine, and piperazine.

As used herein, "isolated" means that a compound is substantially free of solvents and/or impurities.

In particular, with regards to intermediates (i.e. compounds other than compounds of Formula I or Compound A), "isolated" means substantially free of related impurities (e.g. impurities containing a substructure of the isolated intermediate whose molecular mass is at least 50% of the molecular mass of the intermediate) with no regard to solvents or non-related impurities such as inorganics, where "substantially free of related impurities" means that related impurities are together less than 20% of the weight of the intermediate. Thus, in some examples, the solvent content for isolated intermediates can be over 50 wt% when an intermediate is carried into the next step. Likewise, in some eexamplesthe inorganics content for isolated intermediates can also be over 50 wt% when an intermediate is carried into the next step.

Similarly, with regards to compounds of Formula I (e.g. Compound A), "isolated" means substantially free of all related and non-related impurities, solvents, and inorganics, where "substantially free of all related and non-related impurities, solvents, and inorganics" means that related impurities are together less than 3% by weight of the total weight of the obtained mass (including target compound, solvent, etc.), non-related impurities are together less than 3% by weight of the total weight of the obtained mass (including target compound, solvent, etc.), solvents (other than water) are less than 5% of the total weight of the obtained mass (including target compound, solvent, etc.), and inorganics are less than 3% by weight of the total weight of the obtained mass (including target compound, solvent, etc.). Thus, in some examples, water is often present at up to 5wt% in isolated compounds of Formula I (e.g. Compound A). Similarly, in some examples, solvents are less than 0.5 wt% in isolated Compound A. In some examples Absolute purity on a water and solvent corrected basis is usually above 98 wt% in isolated compounds of Formula I (e.g. Compound A).

The term "hydrate" as used herein with reference to an aldehyde (e.g. "an aldehyde or hydrate thereof") refers to the hydrate form of the aldehyde: 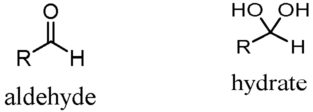 In particular, in some examples, when an aldehyde is contacted with another compound (e.g. in a chemical reaction), the aldehyde can originally be in its hydrate form, which can, for example have better handling properties than the aldehyde form (e.g. chloral hydrate, Cl₃CH(OH)₂, can be easier to handle that chloral itself, Cl₃CHO, because chloral hydrate is crystalline and not subject to the possible polymerization that chloral can be subject to). Accordingly, even if the species in a chemical reaction that reacts with the compound it is contacted with is an aldehyde, the original reagent used in the reaction can be the hydrate form of the aldehyde which can be in equilibrium with the aldehyde form (and said equilibrium can be shifted towards the aldehyde by removing water, for example using molecular sieves, co-distillation with acetonitrile or other solvents, and/or other water removal methods identifiable to a skilled person).

The term "contacting" as used herein refers to bringing two or more substances (e.g. compounds) into sufficiently close proximity such that chemical reaction of the molecules of the two substances can occur. In some examples, the contacting of the two substances occurs when the two substances are dissolved in a solvent. In some examples, a first substance is contacted with a second substance by dissolving and/or suspending the first substance in a solvent (e.g. a liquid solvent) and either i) adding the second substance (e.g. neat or in a solvent such as a liquid solvent) to the solution or suspension of the first substance, or ii) adding the solution or suspension of the first substance to the second substance (which itself could be dissolved and/or suspended in a solvent) thereby allowing the molecules of the two substances to react.

The compounds of the disclosure may contain one or more chiral centers and/or double bonds and, therefore, exist as stereoisomers, such as geometric isomers, enantiomers or diastereomers. The term "stereoisomers" when used herein consist of all geometric isomers, enantiomers or diastereomers. These compounds may be designated by the symbols "R" or "S," depending on the configuration of substituents around the stereogenic carbon atom. The present invention encompasses various stereoisomers of these compounds and mixtures thereof. Stereoisomers include enantiomers and diastereomers. Mixtures of enantiomers or diastereomers may be designated "(.+ -.)", or "+/-", or "±" in nomenclature, but the skilled artisan will recognize that a structure may denote a chiral center implicitly.

Individual enantiomers and diastereoisomers of compounds of the present disclosure can be prepared synthetically from commercially available starting materials that contain asymmetric or stereogenic centers, or by preparation of racemic mixtures followed by resolution methods well known to those of ordinary skill in the art. These methods of resolution are exemplified by (1) attachment of a mixture of enantiomers to a chiral auxiliary, separation of the resulting mixture of diastereomers by recrystallization or chromatography and liberation of the optically pure product from the auxiliary, (2) salt formation employing an optically active resolving agent, (3) direct separation of the mixture of optical enantiomers on chiral liquid chromatographic columns or (4) kinetic resolution using stereoselective chemical or enzymatic reagents. Racemic mixtures can also be resolved into their component enantiomers by well-known methods, such as chiral-phase gas chromatography or crystallizing the compound in a chiral solvent. Stereoselective syntheses, a chemical or enzymatic reaction in which a single reactant forms an unequal mixture of stereoisomers during the creation of a new stereocenter or during the transformation of a pre-existing one, are well known in the art. Stereoselective syntheses encompass both enantio- and diastereoselective transformations. For examples, see Carreira and Kvaemo, Classics in Stereoselective Synthesis, Wiley-VCH: Weinheim, 2009.

Where a range of values is provided, it is intended that each intervening value between the upper and lower limit of that range and any other stated or intervening value in that stated range is encompassed within the disclosure. For example, if a range of 1 µm to 8 µm is stated, it is intended that 2 µm, 3 µm, 4 µm, 5 µm, 6 µm, and 7 µm are also explicitly disclosed, as well as the range of values greater than or equal to 1 µm and the range of values less than or equal to 8 µm. Similarly, if a range such as C₁-C₆ alkyl is stated, it is intended that C₁ alkyl, C₂ alkyl, C₃ alkyl, C₄ alkyl, C₅ alkyl, and C₆ alkyl are also explicitly disclosed.

The close-ended term "consist essentially of" or "consist of" is within the scope of the openended term "comprising," "containing," or "including".

### II. Processes

### A. Preparation of Compound of Formula I

An exemplary process of producing a compound of formula I or a pharmaceutically acceptable salt thereof comprises contacting a compound of formula II, as defined above, and a coupling agent in an organic solvent to obtain a compound of formula I or a pharmaceutically acceptable salt thereof, wherein the coupling agent comprises an art recognized agent for coupling an amine and carboxylic acid to form an amide bond:

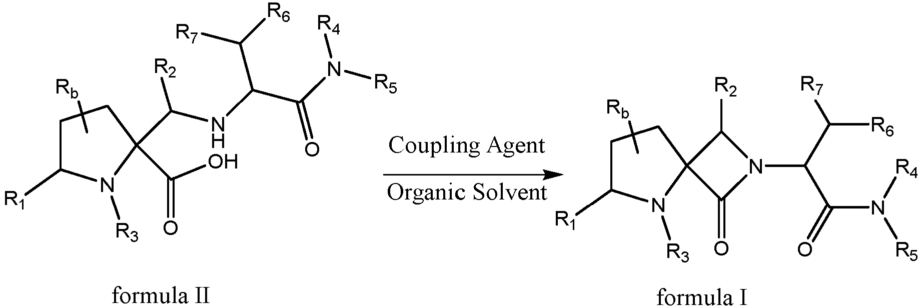

In some examples, the coupling agent comprises a carbodiimide optionally together with 1-hydroxybenzotriazole (HOBt). In some examples, the coupling agent comprises a carbodiimide selected from dicyclohexylcarbodiimide (DCC), diisopropylcarbodiimide (DIC), and ethyl-(N',N'-dimethylamino)propylcarbodiimide hydrochloride (EDC).

In some examples, the coupling agent comprises (Benzotriazol-1-yloxy)tris(dimethylamino)phosphonium hexafluorophosphate (BOP), (Benzotriazol-1-yloxy)tripyrrolidinophosphonium hexafluorophosphate (PyBOP), (7-Azabenzotriazol-1-yloxy)tripyrrolidinophosphonium hexafluorophosphate (PyAOP), Bromotripyrrolidinophosphonium hexafluorophosphate (PyBrOP), Bis(2-oxo-3-oxazolidinyl)phosphinic chloride (BOP-Cl), O-(Benzotriazol-1-yl)-N,N,N',N'-tetramethyluronium hexafluorophosphate (HBTU), O-(Benzotriazol-1-yl)- N,N,N',N'-tetramethyluronium tetrafluoroborate (TBTU), O-(7-Azabenzotriazol-1-yl)-N,N,N',N'-tetramethyluronium hexafluorophosphate (HATU), O-(7-Azabenzotriazol-1-yl)- N,N,N',N'-tetramethyluronium tetrafluoroborate (TATU), O-(6-Chlorobenzotriazol-1-yl)-N,N,N',N'-tetramethyluronium hexafluorophosphate (HCTU), O-[(Ethoxycarbonyl)cyanomethylenamino]-N,N,N',N'-tetramethyluronium tetrafluoroborate (TOTU), (1-Cyano-2-ethoxy-2-oxoethylidenaminooxy)dimethylamino-morpholino-carbenium hexafluorophosphate (COMU), O-(N-Succinimidyl)-1,1,3,3-tetramethyl-uronium tetrafluoroborate (TSTU), O-(5-Norbornene-2,3-dicarboximido)-N,N,N',N'-tetramethyluronium tetrafluoroborate (TNTU), O-(1,2-Dihydro-2-oxo-1-pyridyl-N,N,N',N'-tetramethyluronium tetrafluoroborate (TPTU), N,N,N',N'-Tetramethyl-O-(3,4-dihydro-4-oxo-1,2,3-benzotriazin-3-yl)uranium tetrafluoroborate (TDBTU), N,N,N',N'-Tetramethylchloroformamidinium Hexafluorophosphate (TCFH), 3-(Diethylphosphoryloxy)-1,2,3-benzotriazin-4(3H)-one (DEPBT), or Carbonyldiimidazole (CDI).

In some examples, the coupling agent is a di-C₁-C₆ alkyl halophosphate (such as a di-C₁-C₆ alkyl chlorophosphate), a dialkyl phosphinic halide, or propanephosphonic acid anhydride (e.g. T3P^{®}). In some examples, the coupling agent comprises diethyl chlorophosphate.

In some examples, the coupling agent is used together with an organic base. In some examples, the organic base comprises an amine. In some examples, the organic base comprises triethyl amine, N,N-Diisopropylethylamine (DIPEA), or 4-Dimethylaminopyridine (DMAP). In some examples, the organic base comprises triethyl amine.

In some examples, the process is carried out in an organic solvent comprising dimethylformamide (DMF), dimethyl sulfoxide (DMSO), N-Methyl-2-pyrrolidone (NMP), acetonitrile (MeCN), dichloromethane (DCM), tetrahydrofuran (THF), or 2-methyltetrahydrofuran (2-MeTHF). In some examples, the solvent comprises THF.

In some examples, the process further comprises contacting a compound of formula IV with a base in a solvent to obtain a compound of formula III, wherein the compound of formula III is subsequently subjected to alkylating agent (such as alkyl halide) or carbamating agent (such as di-R₈ dicarbonate, for example BOC₂O) to obtain a compound of formula II, wherein R_{b}, R₁, R₂, R₃, R₄, R₅, R₆, and R₇ are the same as described above, and R₉ is -CCl₃:

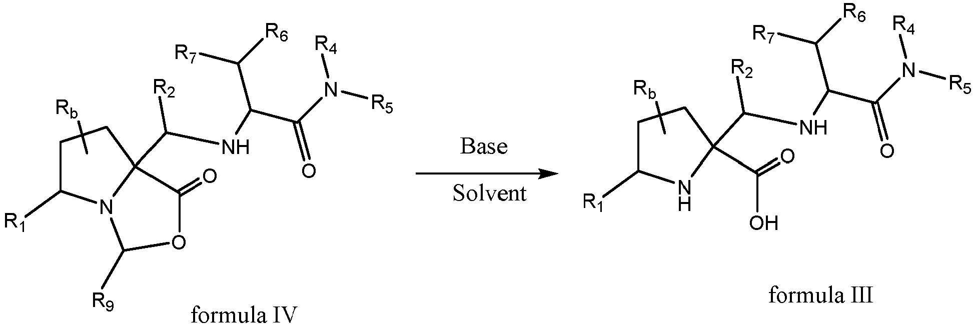

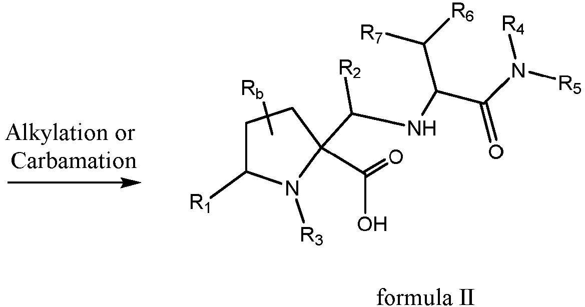

In some examples, the base comprises an amine. In some examples, the base comprises an amine selected from triethyl amine or DIPEA.

In some examples, the solvent is an aqueous solvent. In some examples, the aqueous solvent is a mixture of water with acetonitrile or a mixture of water with THF (or with 2-MeTHF). In other examples, the aqueous solvent is a mixture of water with methanol, a mixture of water with ethanol, a mixture of water with isopropanol, a mixture of water with DMF, or a mixture of water with DMSO.

In some examples, the process further comprises contacting a compound of formula VII with an amine of formula VIII to obtain a condensed product of formula V or VI, which is subsequently contacted with a reducing compound to obtain a compound of formula IV, wherein R_{b}, R₁, R₂, R₃, R₄, R₅, R₆, R₇, and R₉ are the same as described above:

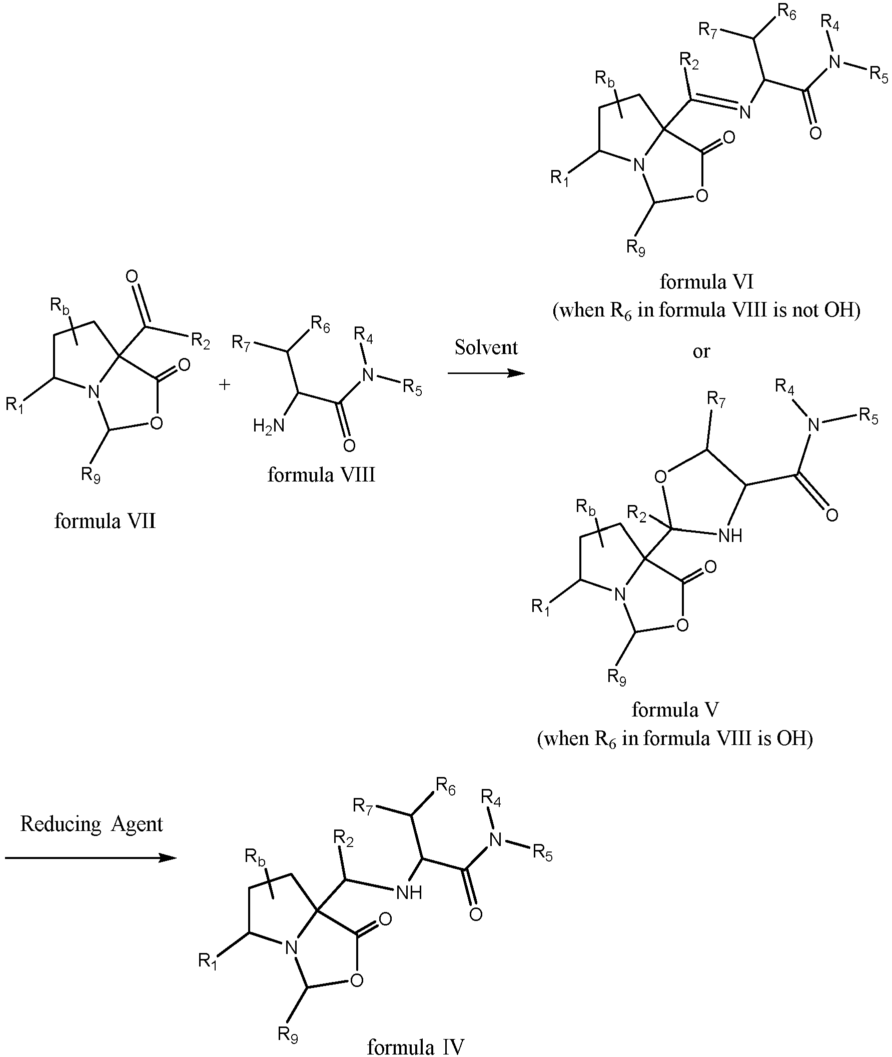

In some examples, the solvent for the condensation reaction comprises dichloromethane. In some examples, the solvent for the condensation reaction comprises toluene. In some examples, the solvent comprises DMF. In some examples, the solvent comprises THF. In some examples, the solvent comprises 2-MeTHF. In some examples, the solvent comprises DMF. In some examples, the solvent comprises acetonitrile.

In some examples, the reducing agent comprises a boron hydride. In some examples, the reducing agent comprises a boron hydride selected from sodium borohydride, lithium borohydride, calcium borohydride, magnesium borohydride, sodium triacetoxyborohydride (NaBH(OAc)₃), NBu₄BH₄, NaCNBH₃, and NMe₄BH(OAc)₃. In some examples, the reducing agent comprises sodium triacetoxyborohydride (NaBH(OAc)₃).

In some examples, before contacting it with the reducing agent, the compound of formula V or VI is not isolated.

In some examples, the process further comprises contacting the compound of formula IX with a base known in the art to deprotonate the α carbon next to a carbonyl (CO) in a solvent (e.g. lithium diisopropylamide (LDA), potassium tert-butoxide (KOtBu), and others identifiable to a skilled person upon a reading of the present disclosure), which is subsequently followed by addition of R₂C(O)OR (R is methyl, ethyl, phenyl, -CH₂CF₃, or -CH₂CN) to obtain the compound of formula VII:

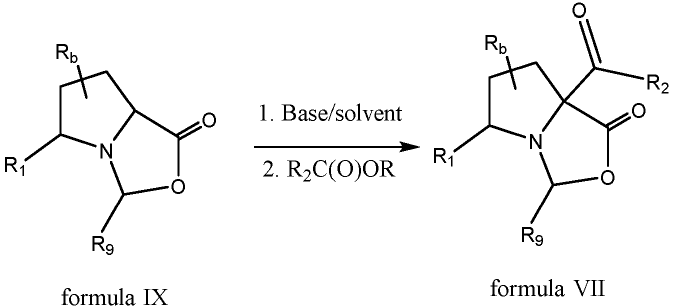

In some examples, the deprotonation is conducted at a temperature below 0 °C, such as at a temperature of -80 to -20 °C.

In some examples, the base comprises lithium diisopropylamide. In some examples, the base is potassium tert-butoxide.

In some examples, the solvent comprises toluene with methyl *tert-butyl* ether (MTBE) optionally added. In some examples, the solvent is MTBE. In some examples, the solvent comprises THF, optionally mixed with toluene or MTBE. In some examples, the solvent comprises 2-MeTHF, optionally mixed with toluene or MTBE.

In some examples, R₂C(O)OR is added to the deprotonated formula IX at a temperature below 0 °C, such as at a temperature of -80 to -20 °C, for example about -60 °C.

In some examples, the process further comprises contacting a compound of formula X with an aldehyde R₉CHO, or a hydrate thereof, to obtain a compound of formula IX:

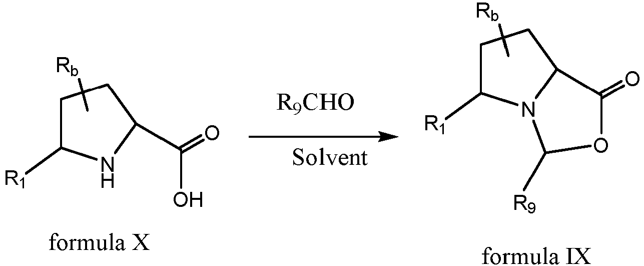

In some examples, the solvent used to obtain the compound of formula IX comprises THF, 2-MeTHF, acetonitrile, toluene, dichloromethane, chloroform, or MTBE.

In some examples, the compound of formula I is a compound of formula Ia:

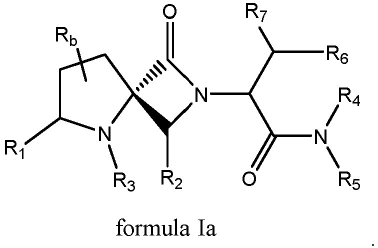

In some examples, the compound of formula Ia is a compound of formula Ib:

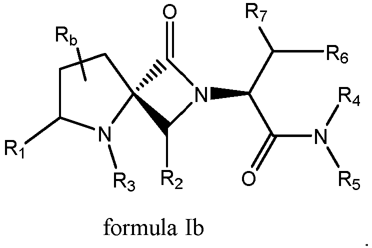

In some examples, the compound of formula Ib is a compound of formula Ic:

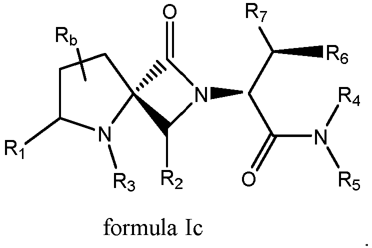

In some examples, the compound of formula II is a compound of formula IIa:

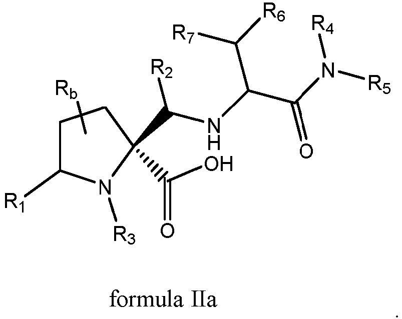

In some examples, the compound of formula IIa is a compound of formula IIb:

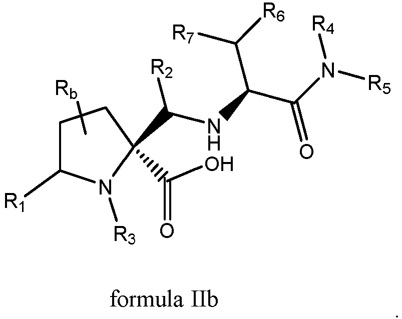

In some examples, the compound of formula IIa is a compound of formula IIc:

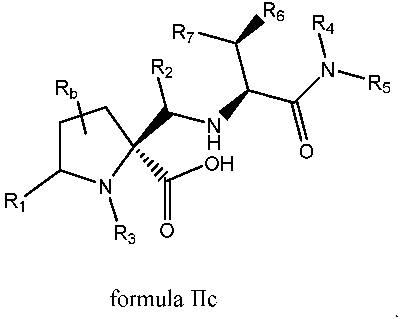

In some examples, the compound of formula III is a compound of formula IIIa:

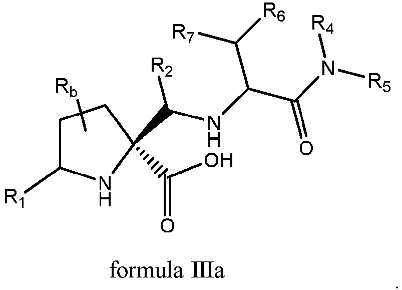

In some examples, the compound of formula IIIa is a compound of formula IIIb:

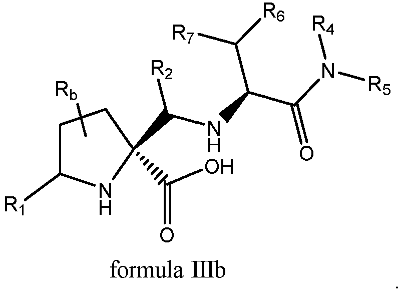

In some examples, the compound of formula IIIa is a compound of formula IIIc:

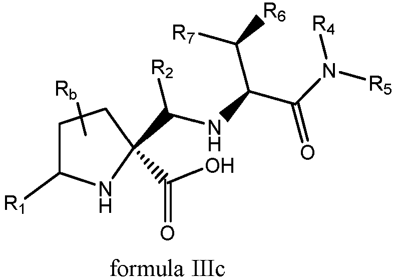

In some examples, the compound of formula IV is a compound of formula IVa:

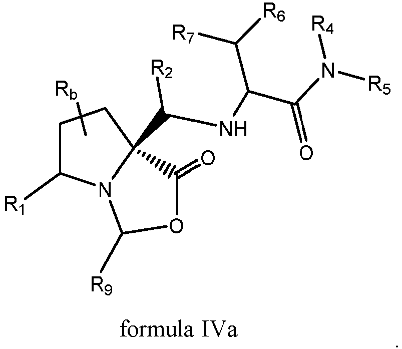

In some examples, the compound of formula IVa is a compound of formula IVb:

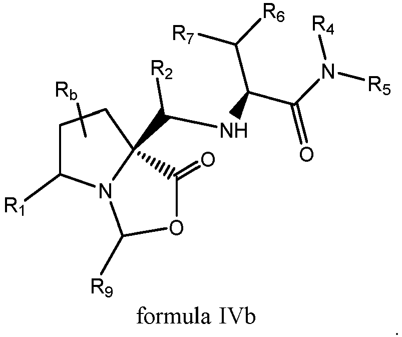

In some examples, the compound of formula IVb is a compound of formula IVc:

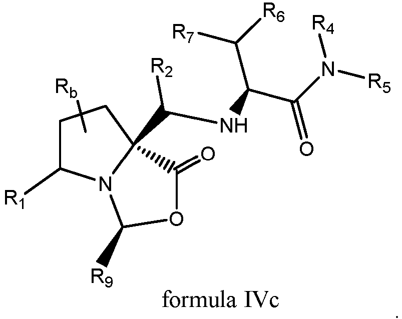

In some examples, the compound of formula IVb is a compound of formula IVd:

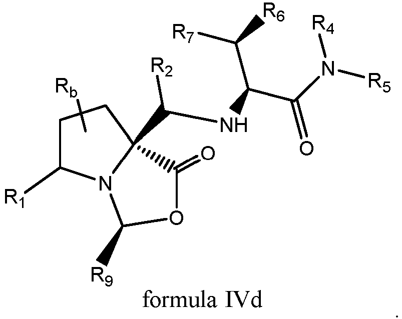

In some examples, the compound of formula V and the compound of formula VI are a compound of formula Va and a compound of formula VIa, respectively:

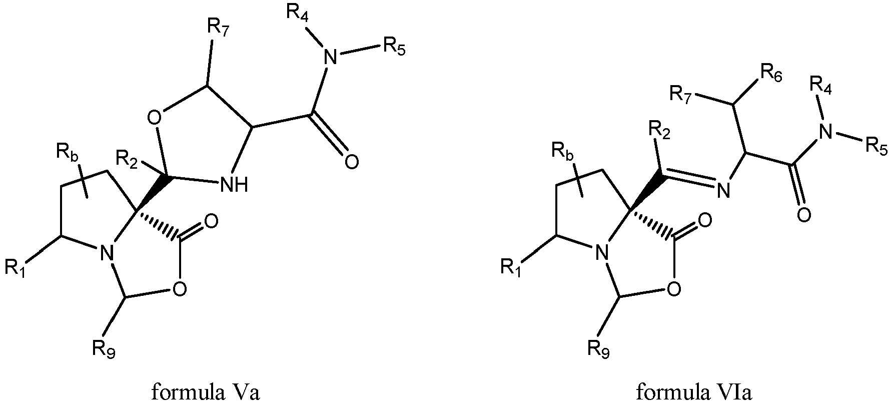

In some examples, the compound of formula Va and the compound of formula VIa are a compound of formula Vb and a compound of formula VIb, respectively:

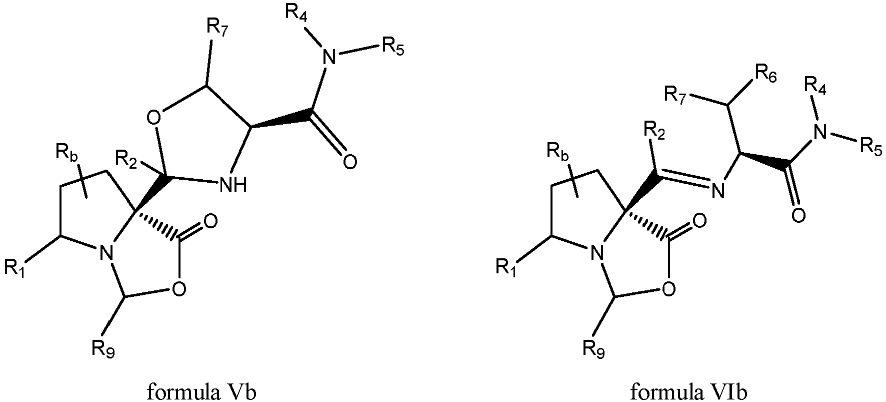

In some examples, the compound of formula Vb and the compound of formula VIb are a compound of formula Vc and a compound of formula Vic, respectively:

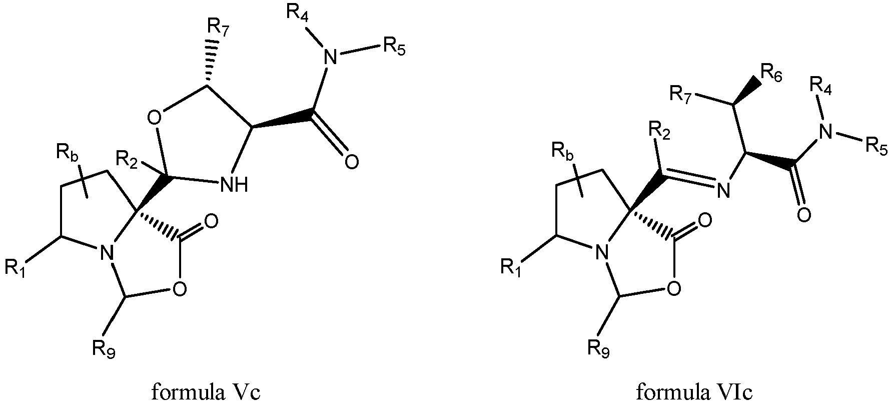

In some examples, the compound of formula Vc and the compound of formula VIc are a compound of formula Vd and a compound of VId, respectively:

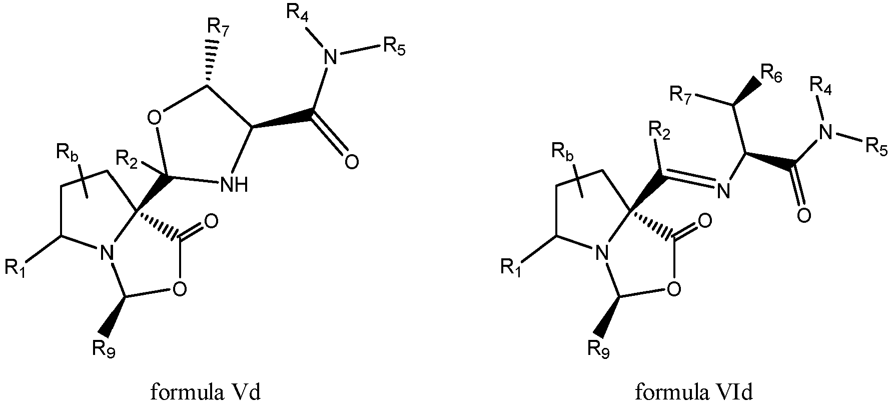

In some examples, the compound of formula VII is a compound of formula VIIa:

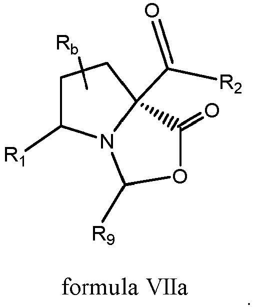

In some examples, the compound of formula VIIa is a compound of formula VIIb:

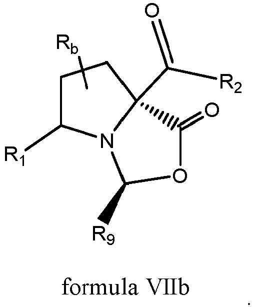

In some examples, the compound of formula VIII is a compound of formula VIIIa:

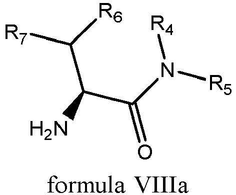

In some examples, the compound of formula VIIIa is a compound of formula VIIIb:

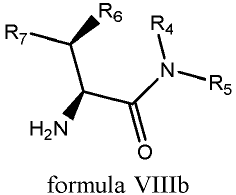

In some examples, the compound of formula IX is a compound of formula IXa:

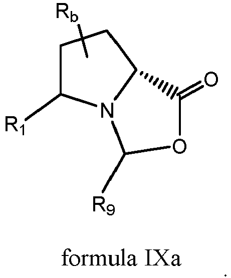

In some examples, the compound of formula IXa is a compound of formula IXb:

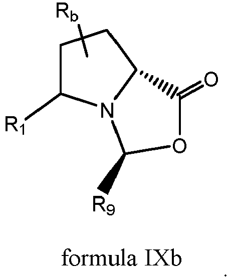

In some examples, the compound of formula X is a compound of formula Xa:

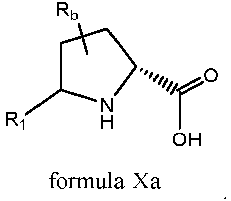

In some examples, in each of formulae I to X (including formulae thereof wherein the chiral center(s) is defined), where applicable,
R_{b} is selected from H, halogen, cyano and C₁-C₆ alkyl;
R₁ is H or C₁-C₆ alkyl;
R₂ is H or C₁-C₆ alkyl;
R₃ is selected from C₁-C₆ alkyl and C(O)OR₈;
R₈ is selected from C₁-C₆ alkyl, C₁-C₆ haloalkyl, C₂-C₆ alkenyl, C₂-C₆ alkynyl, C₃-C₁₀ cycloalkyl, and -CH₂-C₃-C₁₀ cycloalkyl, wherein C₃-C₁₀ cycloalkyl is optionally substituted with one, two, or three groups selected from C₁-C₃ alkyl;
R₄ and R₅ are independently selected from H and C₁-C₆ alkyl; or R₄ and R₅ together with the nitrogen to which they are attached form heterocyclyl including from 4 to 6 ring atoms; wherein the heterocyclyl includes not more than two ring heteroatoms (including the nitrogen atom attached to R₄ and R₅), and the second ring heteroatom, when present, is independently selected from N-C₁-C₆ alkyl, O and S; and wherein the heterocyclyl is optionally substituted with one, two, or three substituents independently selected from halogen, cyano, C₁-C₆ alkyl, and C₁-C₆ alkoxy;
R₆ is selected from -OH, C₁-C₆ alkoxy, -OC(O)-C₁-C₆ alkyl, and -OC(O)phenyl;
R₇ is H or C₁-C₆ alkyl; and
R₉ is selected from CCl₃.

In some examples, in each of formulae I to X (including formulae thereof wherein the chiral center(s) is defined), where applicable, R₁ and R₂ are H.

In some examples, in each of formulae I to X (including formulae thereof wherein the chiral center(s) is defined), where applicable, each of R₁, R₂, R₄, and R₅ is H.

In some examples, in each of formulae I to VIII (including formulae thereof wherein the chiral center(s) is defined), R₆ is OH.

In some examples, in each of formulae I to VIII (including formulae thereof wherein the chiral center(s) is defined), R₇ is C₁-C₆ alkyl, such as methyl.

In some examples, in each of formula I and II (including formulae thereof wherein the chiral center(s) is defined), R₃ is OC(O)R₈, wherein R₈ is C₁-C₆ alkyl, such as tertiary butyl.

In some examples, the compound of formula II is selected from the following compounds:

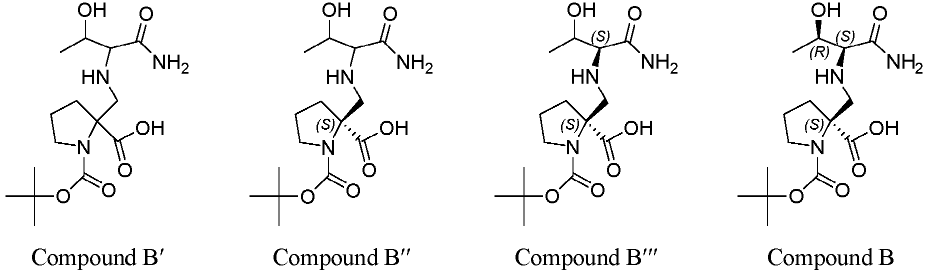

In some examples, the compound of formula III is selected from the following compounds:

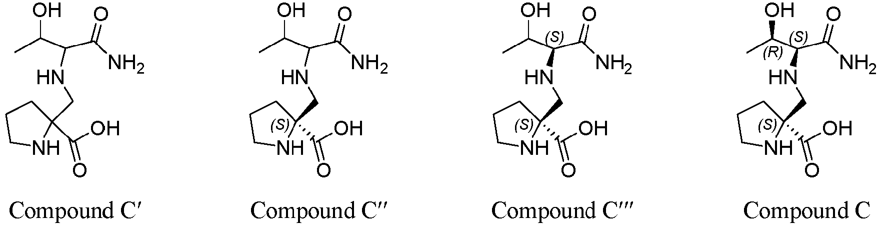

In some examples, the compound of formula IV is selected from the following compounds:

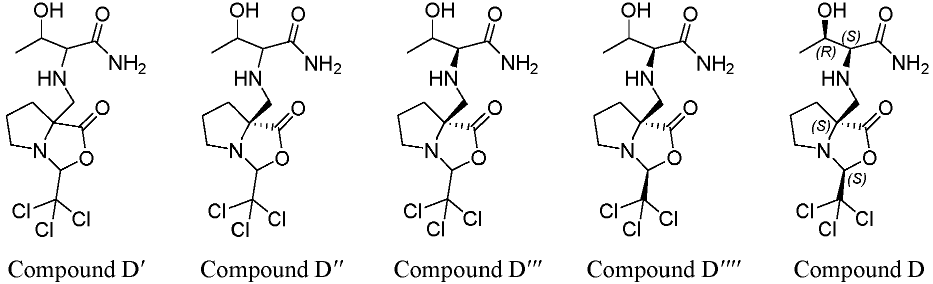

In some examples, the compound of formula V is selected from the following compounds:

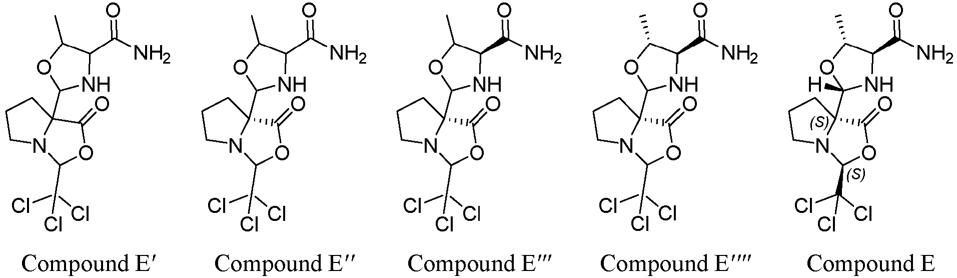

### B. Preparation of Compound A

In another example, a process of producing *tert*-butyl (*S*)-2-((2*S*,3*R*)-1-amino-3-hydroxy-1-oxobutan-2-yl)-1-oxo-2,5-diazaspiro[3.4]octane-5-carboxylate ("Compound A") comprises contacting Compound B with a coupling agent in an organic solvent to obtain Compound A, wherein the coupling agent comprises an art recognized agent for coupling an amine and carboxylic acid to form an amide bond: 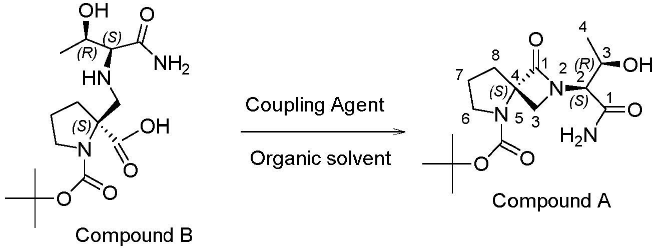 and wherein Compound B is obtained by contacting Compound C with di-tert-butyl dicarbonate (BOC₂O) in a solvent:

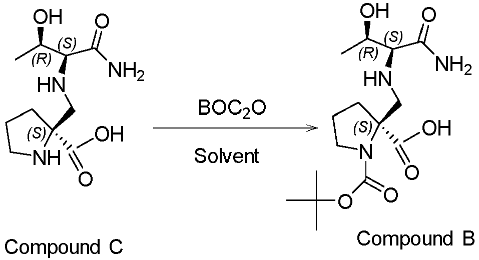

In some examples, the coupling agent comprises a carbodiimide optionally together with 1-hydroxybenzotriazole (HOBt). In some examples, the coupling agent comprises a carbodiimide selected from dicyclohexylcarbodiimide (DCC), diisopropylcarbodiimide (DIC), and ethyl-(N',N'-dimethylamino)propylcarbodiimide hydrochloride (EDC).

In some examples, the coupling agent comprises (Benzotriazol-1-yloxy)tris(dimethylamino)phosphonium hexafluorophosphate (BOP), (Benzotriazol-1-yloxy)tripyrrolidinophosphonium hexafluorophosphate (PyBOP), (7-Azabenzotriazol-1-yloxy)tripyrrolidinophosphonium hexafluorophosphate (PyAOP), Bromotripyrrolidinophosphonium hexafluorophosphate (PyBrOP), Bis(2-oxo-3-oxazolidinyl)phosphinic chloride (BOP-Cl), O-(Benzotriazol-1-yl)-N,N,N',N'-tetramethyluronium hexafluorophosphate (HBTU), O-(Benzotriazol-1-yl)- N,N,N',N'-tetramethyluronium tetrafluoroborate (TBTU), O-(7-Azabenzotriazol-1-yl)-N,N,N',N'-tetramethyluronium hexafluorophosphate (HATU), O-(7-Azabenzotriazol-1-yl)- N,N,N',N'-tetramethyluronium tetrafluoroborate (TATU), O-(6-Chlorobenzotriazol-1-yl)-N,N,N',N'-tetramethyluronium hexafluorophosphate (HCTU), O-[(Ethoxycarbonyl)cyanomethylenamino]-N,N,N',N'-tetra methyluronium tetrafluoroborate (TOTU), (1-Cyano-2-ethoxy-2-oxoethylidenaminooxy)dimethylamino-morpholino-carbenium hexafluorophosphate (COMU), O-(N-Suc-cinimidyl)-1,1,3,3-tetramethyl-uronium tetrafluoroborate (TSTU), O-(5-Norbornene-2,3-dicarboximido)-N,N,N',N'-tetramethyluronium tetrafluoroborate (TNTU), O-(1,2-Dihydro-2-oxo-1-pyridyl-N,N,N',N'-tetramethyluronium tetrafluoroborate (TPTU), N,N,N',N'-Tetramethyl-O-(3,4-dihydro-4-oxo-1,2,3-benzotriazin-3-yl)uranium tetrafluoroborate (TDBTU), N,N,N',N'-Tetramethylchloroformamidinium Hexafluorophosphate (TCFH), 3-(Diethylphosphoryloxy)-1,2,3-benzotriazin-4(3H)-one (DEPBT), or Carbonyldiimidazole (CDI).

In some examples, the coupling agent is a di-C₁-C₆ alkyl halophosphate (such as a di-C₁-C₆ alkyl chlorophosphate), a dialkyl phosphinic halide, or propanephosphonic acid anhydride (e.g. T3P^{®}). In some examples, the coupling agent comprises diethyl chlorophosphate.

In some examples, the coupling agent is used together with an organic base. In some examples, the organic base comprises an amine. In some examples, the organic base comprises triethyl amine, N,N-Diisopropylethylamine (DIPEA), or 4-Dimethylaminopyridine (DMAP). In some examples, the organic base comprises triethyl amine.

In some examples, the process is carried out in an organic solvent comprising dimethylformamide (DMF), dimethyl sulfoxide (DMSO), N-Methyl-2-pyrrolidone (NMP), acetonitrile (MeCN), dichloromethane (DCM), tetrahydrofuran (THF), or 2-methyltetrahydrofuran (2-MeTHF). In some examples, the solvent comprises THF.

In some examples, the solvent used to prepare Compound B comprises acetonitrile, methanol, ethanol, isopropanol, MTBE, toluene, acetone, CH₂Cl₂, THF, 2-MeTHF, water, and mixtures thereof. In some examples, an amine, such as triethyl amine is present in the reaction to prepare Compound B.

In some examples, the process further comprises contacting Compound D with a base in a solvent to obtain Compound C:

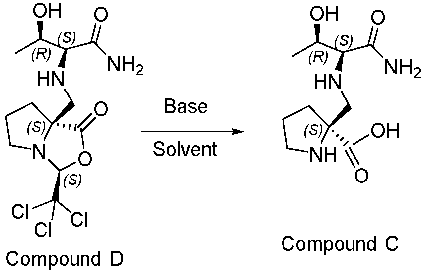

In some examples, the base comprises an amine. In some examples, the base comprises an amine selected from triethyl amine and DIPEA.

In some examples, the solvent is an aqueous solvent. In some examples, the aqueous solvent is a mixture of water with acetonitrile or a mixture of water with THF (or with 2-MeTHF). In other embodiments, the aqueous solvent is a mixture of water with methanol, a mixture of water with ethanol, a mixture of water with isopropanol, a mixture of water with DMF, or a mixture of water with DMSO.

In some examples, the process further comprises contacting Compound F with Compound G to obtain a condensed product Compound E, which is subsequently contacted with a reducing compound to obtain Compound D:

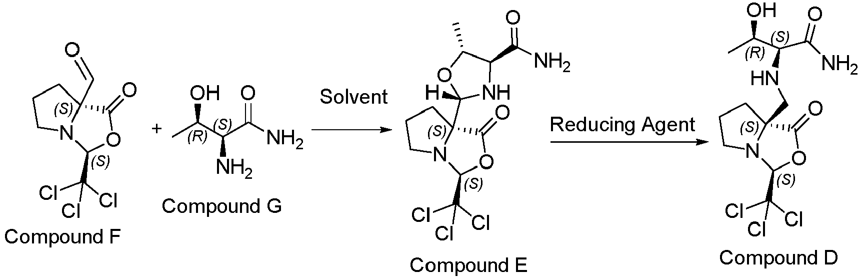

In some examples, the solvent for the condensation reaction comprises dichloromethane. In some examples, the solvent for the condensation reaction comprises toluene. In some examples, the solvent comprises DMF. In some examples, the solvent comprises THF. In some examples, the solvent comprises 2-MeTHF. In some examples, the solvent comprises DMF. In some examples, the solvent comprises acetonitrile.

In some examples, the reducing agent comprises a boron hydride. In some examples, the reducing agent comprises a boron hydride selected from sodium borohydride, lithium borohydride, calcium borohydride, magnesium borohydride, sodium triacetoxyborohydride (NaBH(OAc)₃), NBu₄BH₄, NaCNBH₃, and NMe₄BH(OAc)₃.

In some examples, before contacting it with the reducing agent, Compound E is not isolated.

In some examples, the process further comprises contacting Compound H with a base known in the art to deprotonate the α carbon next to a carbonyl (CO) in a solvent, which is subsequently followed by addition of HC(O)OR (R is methyl, ethyl, phenyl, -CH₂CF₃, or -CH₂CN) to obtain Compound F:

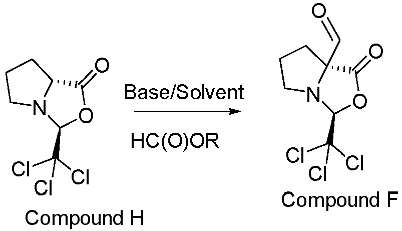

In some examples, the deprotonation is conducted at a temperature below 0 °C, such as at a temperature of -80 to -20 °C, for example about -45 °C.

In some examples, the base comprises lithium diisopropylamide. In some examples, the base is potassium tert-butoxide.

In some examples, the solvent comprises toluene with methyl *tert-butyl* ether (MTBE) optionally added. In some examples, the solvent is MTBE. In some examples, the solvent comprises THF, optionally mixed with toluene or MTBE. In some examples, the solvent comprises 2-MeTHF, optionally mixed with toluene or MTBE.

In some examples, HC(O)OR is added to the deprotonated Compound H at a temperature below 0 °C, such as at a temperature of -80 to -20 °C, for example about -60 °C.

In some examples, the process further comprises contacting D-proline with chloral to obtain Compound H:

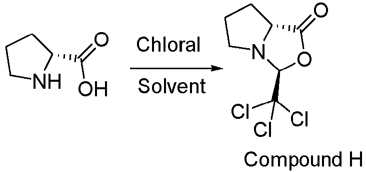

In some examples, the solvent used to obtain Compound H comprises THF, 2-MeTHF, acetonitrile, toluene, dichloromethane, chloroform, or MTBE. In some examples, the solvent used to obtain Compound H comprises acetonitrile.

### III. Intermediates

### A. Compound of Formula II

In one aspect, a compound that is useful in the process of producing the diazaspiro lactam compound of formula I is provided. The compound is a compound of formula II as defined above.

In some embodiments, the compound of formula II is a compound of formula IIa:

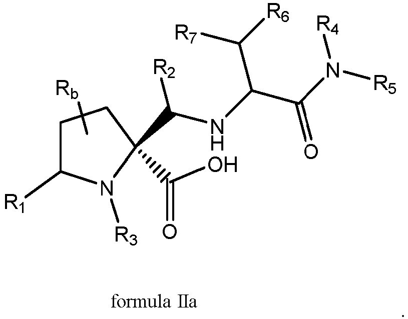

In some embodiments, the compound of formula IIa is a compound of formula IIb:

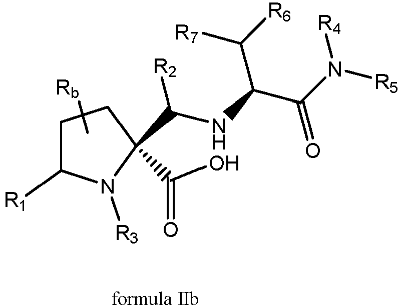

In some embodiments, the compound of formula IIb is a compound of formula IIc:

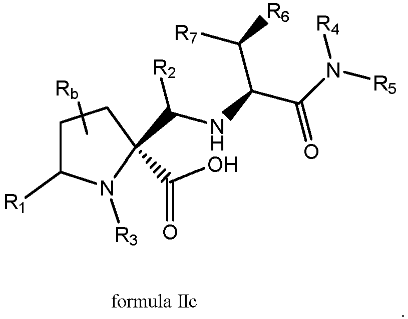

In some embodiments, in each of formulae II, IIa, IIb, and IIc:
R_{b} is selected from H, halogen, cyano and C₁-C₆ alkyl;
R₁ is H or C₁-C₆ alkyl;
R₂ is H or C₁-C₆ alkyl;
R₃ is selected from C₁-C₆ alkyl and C(O)OR₈;
R₈ is selected from C₁-C₆ alkyl, C₁-C₆ haloalkyl, C₂-C₆ alkenyl, C₂-C₆ alkynyl, C₃-C₁₀ cycloalkyl, and -CH₂-C₃-C₁₀ cycloalkyl, wherein C₃-C₁₀ cycloalkyl is optionally substituted with one, two, or three groups selected from C₁-C₃ alkyl;
R₄ and R₅ are independently selected from H and C₁-C₆ alkyl; or R₄ and R₅ together with the nitrogen to which they are attached form heterocyclyl including from 4 to 6 ring atoms; wherein the heterocyclyl includes not more than two ring heteroatoms (including the nitrogen atom attached to R₄ and R₅), and the second ring heteroatom, when present, is independently selected from N-C₁-C₆ alkyl, O and S; and wherein the heterocyclyl is optionally substituted with one, two, or three substituents independently selected from halogen, cyano, C₁-C₆ alkyl, and C₁-C₆ alkoxy;
R₆ is selected from -OH, C₁-C₆ alkoxy, -OC(O)-C₁-C₆ alkyl, and -OC(O)phenyl; and
R₇ is H or C₁-C₆ alkyl.

In some embodiments, in each of formulae II, IIa, IIb, and IIc, R₁ and R₂ are H.

In some embodiments, in each of formulae II, IIa, IIb, and IIc, each of R₁, R₂, R₄, and R₅ is H.

In some embodiments, in each of formulae II, IIa, IIb, and IIc, R₆ is OH.

In some embodiments, in each of formulae II, IIa, IIb, and IIc, R₇ is C₁-C₆ alkyl, such as methyl.

In some embodiments, in each of formulae II, IIa, IIb, and IIc, R₃ is OC(O)R₈, wherein R₈ is C₁-C₆ alkyl, such as tertiary butyl.

In some embodiments, the compound of formula II is selected from the following compounds:

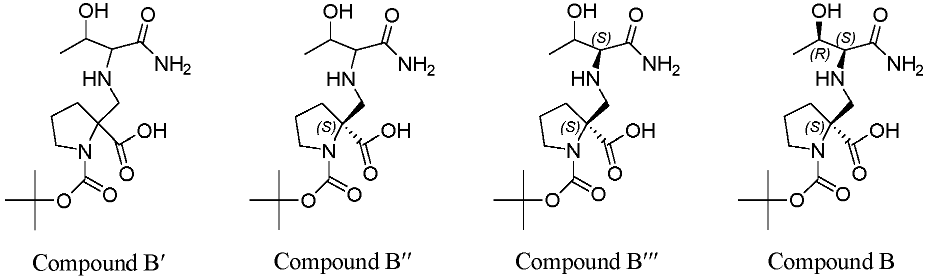

### B. Compound of Formula III

In another aspect, provided is a compound of formula III as defined above:

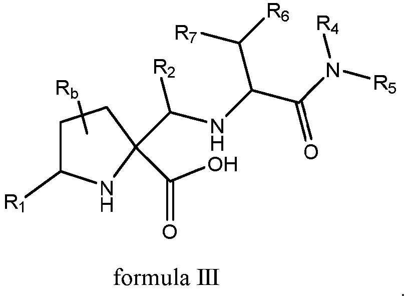

In some embodiments, the compound of formula III is a compound of formula IIIa:

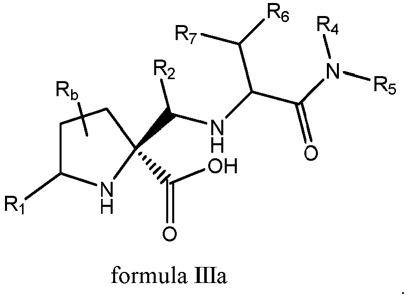

In some embodiments, the compound of formula IIIa is a compound of formula IIIb:

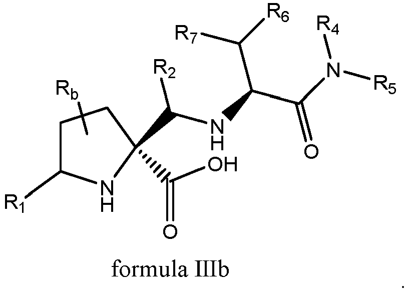

In some embodiments, the compound of formula IIIb is a compound of formula IIIc:

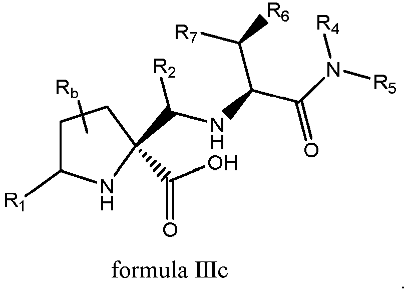

In some embodiments, in each of formulae III, IIIa, IIIb, and IIIc:
R_{b} is selected from H, halogen, cyano and C₁-C₆ alkyl;
R₁ is H or C₁-C₆ alkyl;
R₂ is H or C₁-C₆ alkyl;
R₄ and R₅ are independently selected from H and C₁-C₆ alkyl; or R₄ and R₅ together with the nitrogen to which they are attached form heterocyclyl including from 4 to 6 ring atoms; wherein the heterocyclyl includes not more than two ring heteroatoms (including the nitrogen atom attached to R₄ and R₅), and the second ring heteroatom, when present, is independently selected from N-C₁-C₆ alkyl, O and S; and wherein the heterocyclyl is optionally substituted with one, two, or three substituents independently selected from halogen, cyano, C₁-C₆ alkyl, and C₁-C₆ alkoxy;
R₆ is selected from -OH, C₁-C₆ alkoxy, -OC(O)-C₁-C₆ alkyl, and -OC(O)phenyl; and
R₇ is H or C₁-C₆ alkyl.

In some embodiments, in each of formulae III, IIIa, IIIb, and IIIc, R₁ and R₂ are H.

In some embodiments, in each of formulae III, IIIa, IIIb, and IIIc, each of R₁, R₂, R₄, and R₅ is H.

In some embodiments, in each of formulae III, IIIa, IIIb, and IIIc, R₆ is OH.

In some embodiments, in each of formulae III, IIIa, IIIb, and IIIc, R₇ is C₁-C₆ alkyl, such as methyl.

In some embodiments, the compound of formula III is selected from the following compounds:

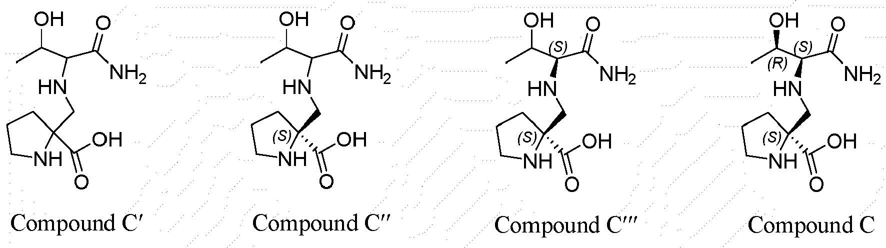

### IV Examples

The following examples are illustrative in nature and are in no way intended to be limiting.

### EXAMPLE 1

### PREPARATION OF COMPOUND OF FORMULA I

The compound of formula I can be prepared by following the following general scheme:

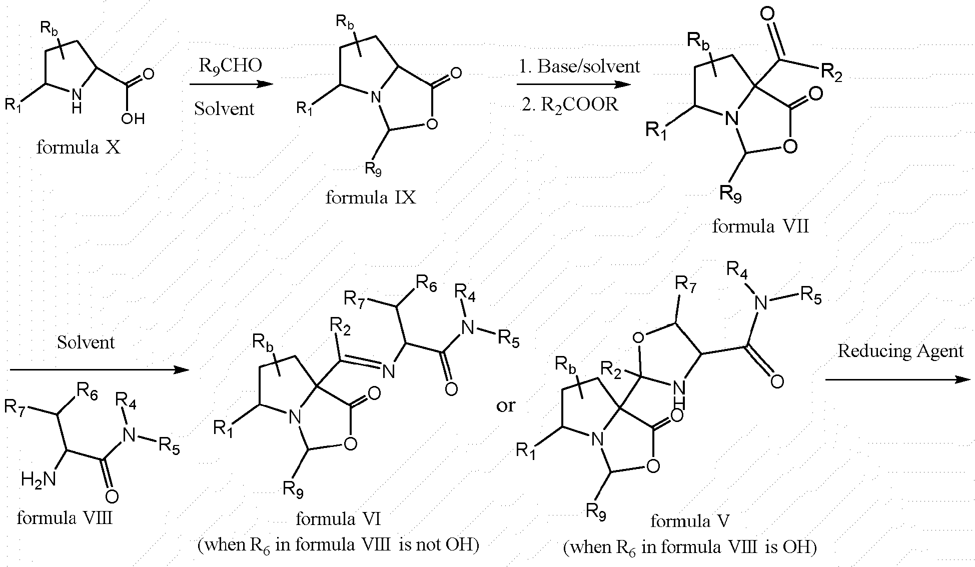

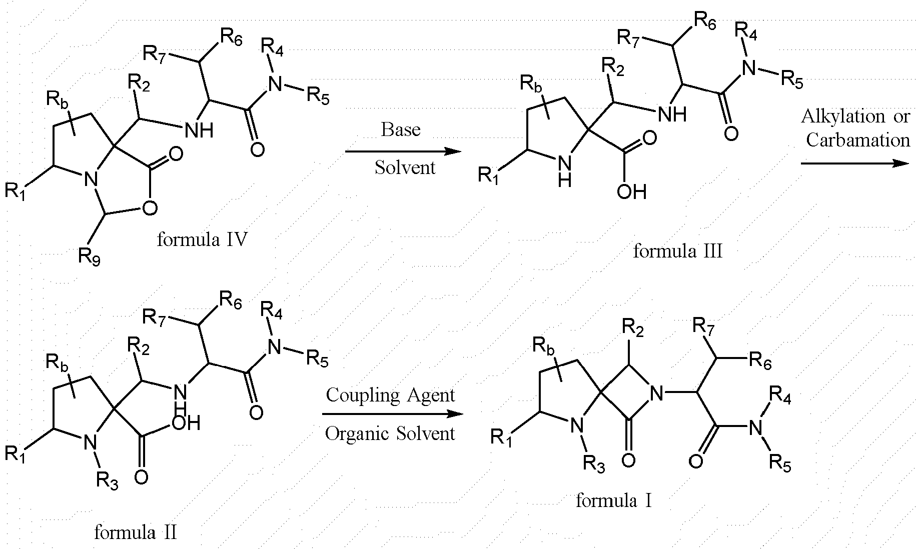

The compound of formula IX is prepared from reacting a proline or derivative thereof (formula X) with an aldehyde R₉CHO, or hydrate thereof. Deprotonating the compound of formula IX with a base such as lithium diisopropylamide or a similar base and subsequent treatment with R₂C(O)OR provides the compound of formula VII. Condensation of the compound of formula VII with a compound of formula VIII gives a compound of formula V (when R₆ in formula VIII is OH) or VI (when R₆ in formula VIII is not OH). Subjecting the compound of formula V or VI to a reducing agent (such as a boron hydride type of reducing agent) produces a compound of formula IV. Treating the compound of formula IV with a basic agent such as an organic amine opens the 5-memebered ring and provides a compound of formula III. Protecting the nitrogen on the 5-membered ring with a dicarbonate or an alkylating agent leads to a compound of formula II. The compound of formula II cyclizes in the presence of a coupling agent to provide the compound of formula I.

Other reaction conditions, such as reaction temperature, solvent, and specific reagent used to perform the reaction can be determined by a skilled artisan.

Individual enantiomers and diasteriomers of compounds of the above formulae can be prepared synthetically from commercially available starting materials that contain asymmetric or stereogenic centers, or by preparation of racemic mixtures followed by resolution methods well known to those of ordinary skill in the art.

### EXAMPLE 2

### PREPARATION OF TERT-BUTYL (S)-2-((2S,3R)-1-AMINO-3-HYDROXY-1-OXOBUTAN-2-YL)-1-OXO-2,5-DIAZASPIRO[3.4]OCTANE-5-CARBOXYLATE ("COMPOUND A")

Compound A was prepared by the following scheme:

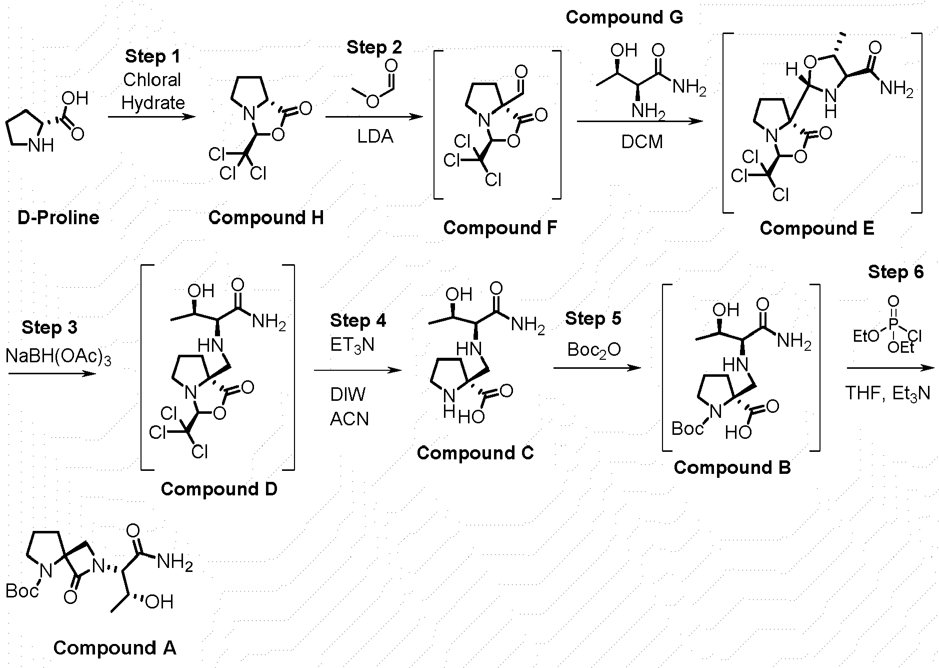

### Step 1: Synthesis of Compound H

A nitrogen purged reactor was charged with acetonitrile, D-proline (69.0 kg), molecular sieves and chloral hydrate (106 kg). The mixture was heated at 50 °C for 5.3 hours. Proton NMR showed complete conversion. The reaction mixture was filtered through a pad of acetonitrile wet Celite and rinsed through with acetonitrile. The filtrate was concentrated to 100 L total volume under vacuum at less than 45°C. N-Butanol (140 L) was added and the mixture was concentrated under vacuum at less than 45°C for 3.5 hours until no further distillate was observed. The mixture was kept at 20 °C overnight then cooled to 0-5 °C and stirred. The precipitate was collected by pressure filtration, then washed with n-butanol. The resultant solid was dried under vacuum at 45°C to afford compound H (108.7 kg, 74.2% yield). ¹H-NMR (DMSO-d₆) δ 1.1-1.4 (m, 1H), 1.4-1.7 (m, 1H), 1.7-2.0 (m, 1H), 2.1-2.5 (m, 1H), 3.2-3.4 (m, 1H), 3.5-3.8 (m, 1H), 4.1-4.4 (m, 1H), 5.8 (s, 1H). MS (ESI) m/z (M-H+2H₂O)⁻ 277.94.

### Step 2: Synthesis of Compound F

A nitrogen purged reactor was charged with toluene, MTBE and Compound H (1eq). The resulting solution was cooled to -55 to -45°C. Lithium diisopropylamide (LDA) solution in THF/n-heptane/ethylbenzene (26.8%, 1.1 eq) was added over 1.3 hour at -50 to -44 °C. The resulting solution was stirred at -45 ± 5 °C for 37 minutes then cooled to -75 to -65°C. A solution of methyl formate (2 eq.) in MTBE was added over 45 minutes at less than -60 °C then rinsed in with MTBE. The mixture was stirred for 44 minutes at -70 to -60°C. A second reactor was flushed with nitrogen and charged with deionized water and citric acid monohydrate. The resulting solution was cooled to 0 to 5°C, and the contents of the first reactor was added over 53 minutes at less than 10 °C and rinsed in with MTBE. The mixture was warmed to 11 °C and the phases separated. The aqueous layer was extracted with MTBE then discarded. The main organic layer then the MTBE wash were washed with a solution of sodium chloride (57%) in water (1.8 vol). The combined organics were concentrated under vacuum at less than 50 °C. Toluene (2×) was added and the mixture concentrated after each addition until the total volume was 47 L. The mixture was cooled to 35 °C and diluted with methylene chloride affording Compound F as a crude solution with a 65.1% yield. Crystallization of a sample of crude compound F from MTBE/ hexanes afforded an analytical sample: ¹H-NMR (DMSO-d₆) δ 1.7-1.8 (m, 1H), 1.8-1.9 (m, 1H), 2.2-2.3 (m, 2H), 3.3-3.4 (m, 1H), 3.5-3.6 (m, 1H), 5.9 (s, 1H), 9.5 (s,1H). MS (ESI) m/z (M+H)⁺ 272.0.

### Step 3: Synthesis of Compound D

A nitrogen purged reactor was charged with crude compound F solution, methylene chloride and compound G (1.2 eq). The resulting suspension was heated to 30-35°C for 6 hours then stirred overnight at 20-25°C. to Compound E. An analytical sample of compound E was isolated via silica gel column chromatography (methylene chloride/ethyl acetate eluent) followed by crystallization from ethyl acetate/ hexanes. ¹H-NMR (DMSO-d₆) δ 1.2 (d, 3H, J = 8 Hz), 1.8-1.9 (m, 2H), 2.0-2.1 (m, 1H), 2.2-2.3 (m, 1H), 3.1-3.2 (m, 1H), 3.2-3.3 (m, 1H), 3.6-3.8 (m, 2H), 4.7 (d, 2H, J = 15 Hz), 5.5 (s,1H), 7.2 (s, 1H), 7.5 (s, 1H). MS (ESI) m/z (M+H)⁺ 372.0.

The crude compound E mixture was cooled to 20 °C and sodium triacetoxyborohydride (3.0 eq) was added over 1.5 H at 20-29 °C then the mixture was stirred 5 hours at 30-35°C.. Water was added at 15-20 °C over 49 min with gas evolution. The media was stirred then the phases separated. The aqueous layer was extracted twice with methylene chloride (2 ×). The combined organics were washed with saturated aqueous sodium bicarbonate. The methylene chloride extracts assayed by HPLC as containing pure compound D with a 78.9% yield. An analytical sample of compound D was crystallized from toluene/ hexane and water. ¹H-NMR (DMSO-d₆) δ 1.1 (d, 3H, J = 8 Hz), 1.8-1.9 (m, 2H), 2.0-2.1 (m, 2H), 2.7-2.8 (m, 2H), 3.1-3.2 (m, 1H), 3.3-3.4 (m, 1H), 3.6-3.7 (m, 1H), 4.7 (d, 2H, J = 6 Hz), 5.6 (s,1H), 7.0 (s, 1H), 7.1 (s, 1H). MS (ESI) m/z (M+H)⁺ 374.1.

### Step 4: Synthesis of Compound C

The crude solution of Compound D was concentrated under vacuum at less than 45 °C to a total volume of 110 L. Acetonitrile was added and the mixture concentrated to a total volume of 110 L. Acetonitrile, water and triethylamine (6 eq) were added and the mixture heated to 45 °C then stirred f . The mixture was concentrated under vacuum at less than 50 °C to a total volume of less than 110 L. Acetonitrile then Isopropanol were added.. The mixture was cooled to 15-20°C, MTBE was added over 1 hour at 15-20°C and the resultant slurry was stirred at 15-20°C and the product collected by filtration. The crude solids were slurried in methanol and stirred at 60-65°C then the suspension was slowly cooled to 20-25°C. The product was collected by filtration and washed with methanol and the solids dried under vacuum at 50 °C to give Compound C with a 72.4% yield. ¹H-NMR (MeOH-d₄) δ 1.23 (3H, d, J = 6.4Hz); 1.9-2.1 (m, 3H), 2.2-2.3 (m, 1H), 2.9 (d, 1H, J = 13Hz), 3.0 (d, 1H, J = 6 Hz), 3.1 (d, 1H, J = 13Hz), 3.2-3.3 (m, 1H), 3.4-3.5 (m, 1H), 3.8 (pentet, 1H, J=6Hz). MS (ESI) m/z (M+H)⁺ 246.2.

### Step 5: Synthesis of Compound B

To a nitrogen purged reactor was charged sequentially acetone, water and Compound C (1 eq). Triethylamine (6 eq) was added to the media in 20 minutes at less than 30 °C and rinsed in with acetone. A solution of di-tert-butyl dicarbonate (1.3 eq) in was added to the mixture at less than 30 °C and rinsed in with acetone (13 L). The mixture was stirred at 20-30°C. n. A solution of *di-tert-*butyl dicarbonate (0.5 eq) in acetone was added to the mixture. A solution of di-tert-butyl dicarbonate (0.5 eq) in acetone was added. The mixture was concentrated at atmospheric pressure to 65 L total volume. Acetone then THF were added and the mixture concentrated to 65 L total volume. The resulting suspension was cooled to 0-5°C then the precipitate collected by filtration and washed with THF and dried under vacuum at 45 °C to afford Compound B with a 90.5% yield. ¹H-NMR (DMSO-d₆) δ 1.1 (d, 3H, J = 6 Hz), 1.3 (s, 5H), 1.4 (s, 4H), 1.7-1.8 (m, 2H), 1.9-2.0 (m, 1H), 2.2-2.4 (m, 1H), 2.5-3.1 (m, 3H), 3.2-3.5 (m, 3H), 3.6-3.7 (m, 1H), 7.2 (d, 1H, J=16Hz), 7.4, (d, 1H, J = 16Hz). MS (ESI) m/z (M+H)⁺ 346.3.

### Step 6: Synthesis of Compound A

To a nitrogen purged reactor was charged THF and Compound B (1 eq). Triethylamine (1.8 eq) was added at 20-25°C and rinsed in with THF. A solution of diethylchlorophosphate (1.8 eq) in THF was added at 20-33°C . After stirring at 25-33°C, a solution of sodium chloride in water was added at 25-30°C and the phases separated. The aqueous layer was extracted twice with ethyl acetate. The combined organics were concentrated under vacuum at less than 60 °C to a total volume of 65 to 70L. Ethyl acetate was added and the mixture concentrated to 65 to 70 L total volume. Ethyl acetate followed by a solution of sodium chloride in water (60 L) were added to the mixture. Phosphoric acid was then added to adjust the pH to 2.0. The mixture was stirred at 20-25°C and the phases separated and the aqueous discarded. The organic layer was washed with a mixture of sodium chloride and aqueous ammonia and the wash back extracted with ethyl acetate. The combined organics were mixed with activated charcoal and stirred overnight then filtered through ethyl acetate wet Celite and rinsed through with ethyl acetate). The filtrate was concentrated under vacuum at less than 60 °C to a total volume of 100 L. Ethyl acetate was added and the mixture concentrated to 100 L after each addition. Ethyl acetate was added and the mixture was cooled to 20-25°C. The mixture was heated to 45-55°C and the residual solids removed by filtration, washed with ethyl acetate and discarded.

The filtrate was concentrated under vacuum at less than 60°C to a total volume of 105 L. The mixture was heated to 65-70°C then cooled to 25 °C. Diisopropyl ether was added and the mixture stirred at 20-25°C. The precipitate was collected by filtration and washed with diisopropyl ether then dried at 50 °C to give Compound A as a white crystalline powder with 67.2% yield. ¹H-NMR (DMSO-d₆) δ 1.1 (m, 3H), 1.3 (s, 4H), 1.4 (s, 5H), 1.7-1.9 (m, 2H), 2.0-2.3 (m, 2H), 3.1-3.5 (m, 3H), 3.5-4.0 (m, 3H), 4.9 (m, 1H), 7.1-7.6 (m, 2H). MS (ESI) m/z (M+Na)⁺ 350.2.

## Claims

1. A compound of formula II: 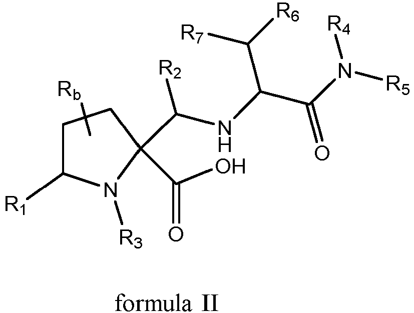 wherein:
R_{b} is selected from H, halogen, cyano and C₁-C₆ alkyl;
R₁ is H or C₁-C₆ alkyl;
R₂ is H or C₁-C₆ alkyl;
R₃ is selected from C₁-C₆ alkyl and C(O)OR₈;
R₈ is selected from C₁-C₆ alkyl, C₁-C₆ haloalkyl, C₂-C₆ alkenyl, C₂-C₆ alkynyl, C₃-C₁₀ cycloalkyl, and -CH₂-C₃-C₁₀ cycloalkyl, wherein C₃-C₁₀cycloalkyl is optionally substituted with one, two, or three groups selected from C₁-C₃ alkyl;
R₄ and R₅ are independently selected from H, C₁-C₆ alkyl, X, and -C₁-C₆ alkylene-X, wherein X is selected from
(i) C₃-C₆ cycloalkyl;
(ii) heteroaryl containing from 5 to 6 ring atoms wherein one, two, or three of the ring atoms are independently selected from N, NH, N(C₁-C₃ alkyl), O, and S;
(iii) heterocyclyl containing from three to six ring atoms wherein 1, 2, or 3 of the ring atoms are independently selected from N, NH, N(C₁-C₃ alkyl), O, and S; and
(iv) phenyl;
wherein C₃-C₆ cycloalkyl, heterocyclyl, heteroaryl, and phenyl are each optionally substituted with one, two, or three substituents independently selected halogen, cyano, C₁-C₆ alkyl, hydroxyl, and C₁-C₆ alkoxy;
or R₄ and R₅ together with the nitrogen to which they are attached form heterocyclyl containing from 4 to 6 ring atoms; wherein the heterocyclyl contains not more than two ring heteroatoms including the nitrogen atom attached to R₄ and R₅, and the second ring heteroatom, when present, is independently selected from N-C₁-C₆ alkyl, O and S; and wherein the heterocyclyl is optionally substituted with one, two, or three substituents independently selected from halogen, cyano, C₁-C₆ alkyl, and C₁-C₆ alkoxy;
R₆ is selected from -OH, C₁-C₆ alkoxy, -OC(O)-C₁-C₆ alkyl, and -OC(O)phenyl; and
R₇ is H or C₁-C₆ alkyl.

2. The compound of claim 1, which is a compound of formula IIa: 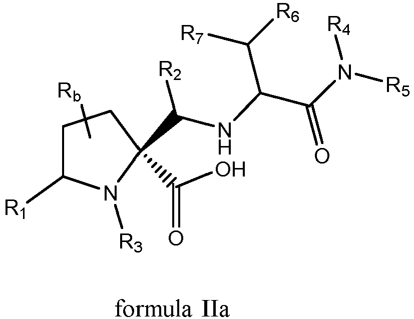 wherein R_{b}, R₁, R₂, R₃, R₄, R₅, R₆, and R₇ are as defined in claim 1.

3. The compound of claim 2, which is a compound of formula IIb: 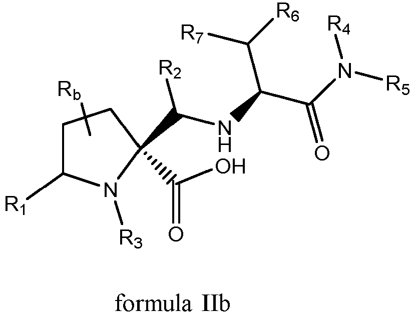 wherein R_{b}, R₁, R₂, R₃, R₄, R₅, R₆, and R₇ are as defined in claim 1.

4. The compound of claim 3, which is a compound of formula IIc: 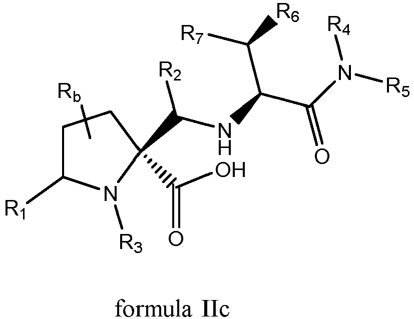 wherein R_{b}, R₁, R₂, R₃, R₄, R₅, R₆, and R₇ are as defined in claim 1.

5. The compound of claim 1, wherein:
R₄ and R₅ are independently selected from H and C₁-C₆ alkyl; or
R₄ and R₅ together with the nitrogen to which they are attached form heterocyclyl containing from 4 to 6 ring atoms; wherein the heterocyclyl contains not more than two ring heteroatoms including the nitrogen atom attached to R₄ and R₅, and the second ring heteroatom, when present, is independently selected from N-C₁-C₆ alkyl, O and S; and wherein the heterocyclyl is optionally substituted with one, two, or three substituents independently selected from halogen, cyano, C₁-C₆ alkyl, and C₁-C₆ alkoxy.

6. The compound of claim 5, wherein each of R₁, R₂, R₄, and R₅ is H.

7. The compound of claim 1, which is selected from the following compounds:

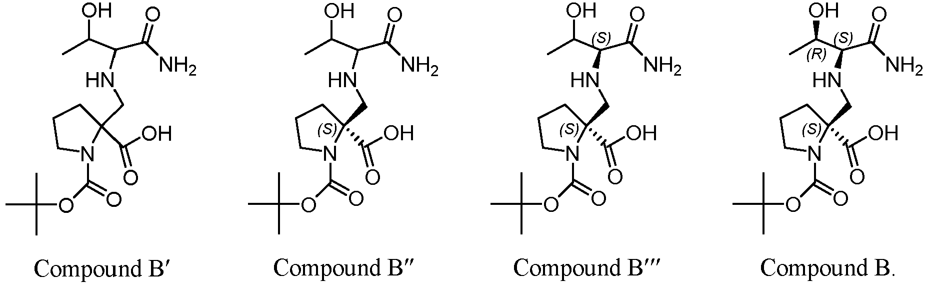

8. The compound of claim 7, which is Compound B.

9. A compound of formula III: 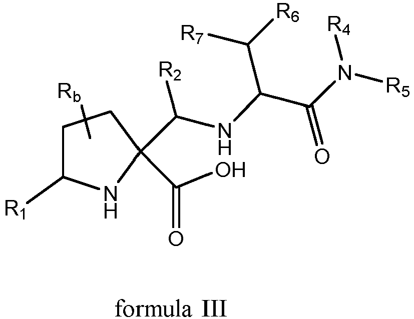 wherein:
R_{b} is selected from H, halogen, cyano and C₁-C₆ alkyl;
R₁ is H or C₁-C₆ alkyl;
R₂ is H or C₁-C₆ alkyl;
R₄ and R₅ are independently selected from H, C₁-C₆ alkyl, X, and -C₁-C₆ alkylene-X, wherein X is selected from
(i) C₃-C₆ cycloalkyl;
(ii) heteroaryl containing from 5 to 6 ring atoms wherein one, two, or three of the ring atoms are independently selected from N, NH, N(C₁-C₃ alkyl), O, and S;
(iii) heterocyclyl containing from three to six ring atoms wherein 1, 2, or 3 of the ring atoms are independently selected from N, NH, N(C₁-C₃ alkyl), O, and S; and
(iv) phenyl;
wherein C₃-C₆ cycloalkyl, heterocyclyl, heteroaryl, and phenyl are each optionally substituted with one, two, or three substituents independently selected halogen, cyano, C₁-C₆ alkyl, hydroxyl, and C₁-C₆ alkoxy;
or R₄ and R₅ together with the nitrogen to which they are attached form heterocyclyl containing from 4 to 6 ring atoms; wherein the heterocyclyl contains not more than two ring heteroatoms including the nitrogen atom attached to R₄ and R₅, and the second ring heteroatom, when present, is independently selected from N-C₁-C₆ alkyl, O and S; and wherein the heterocyclyl is optionally substituted with one, two, or three substituents independently selected from halogen, cyano, C₁-C₆ alkyl, and C₁-C₆ alkoxy;
R₆ is selected from -OH, C₁-C₆ alkoxy, -OC(O)-C₁-C₆ alkyl, and -OC(O)phenyl; and
R₇ is H or C₁-C₆ alkyl.

10. The compound of claim 9, which is a compound of formula IIIa: 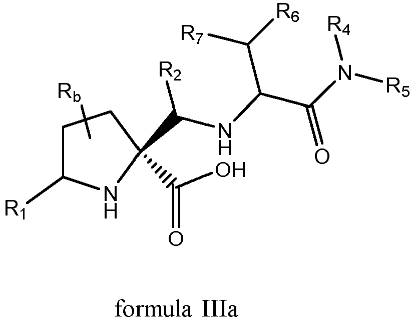 wherein R_{b}, R₁, R₂, R₄, R₅, R₆, and R₇ are as defined in claim 9.

11. The compound of claim 10, which is a compound of formula IIIb: 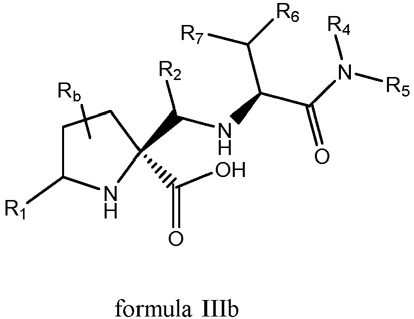 wherein R_{b}, R₁, R₂, R₄, R₅, R₆, and R₇ are as defined in claim 9.

12. The compound of claim 11, which is a compound of formula IIIc: 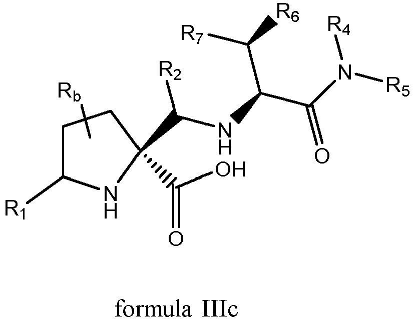 wherein R_{b}, R₁, R₂, R₄, R₅, R₆, and R₇ are as defined in claim 9.

13. The compound of claim 9, wherein:
R₄ and R₅ are independently selected from H and C₁-C₆ alkyl; or
R₄ and R₅ together with the nitrogen to which they are attached form heterocyclyl containing from 4 to 6 ring atoms; wherein the heterocyclyl contains not more than two ring heteroatoms including the nitrogen atom attached to R₄ and R₅, and the second ring heteroatom, when present, is independently selected from N-C₁-C₆ alkyl, O and S; and wherein the heterocyclyl is optionally substituted with one, two, or three substituents independently selected from halogen, cyano, C₁-C₆ alkyl, and C₁-C₆ alkoxy.

14. The compound of claim 13, wherein each of R₁, R₂, R₄, and R₅ is H.

15. The compound of claim 9, which is selected from the following compounds:

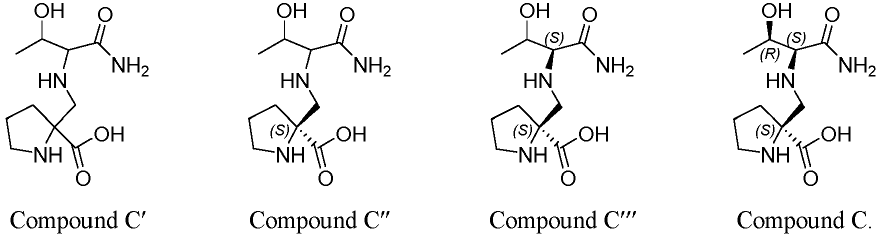

## Patentansprüche

1. Verbindung der Formel II: 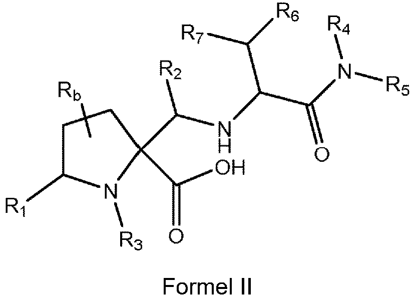 wobei:
R_{b} aus H, Halogen, Cyano und C₁-C₆-Alkyl ausgewählt ist;
R₁ H oder C₁-C₆-Alkyl ist;
R₂ H oder C₁-C₆-Alkyl ist;
R₃ aus C₁-C₆-Alkyl und C(O)OR₈ ausgewählt ist;
R₈ aus C₁-C₆-Alkyl, C₁-C₆-Halogenalkyl, C₂-C₆-Alkenyl, C₂-C₆-Alkynyl, C₃-C₁₀-Cycloalkyl und -CH₂-C₃-C₁₀-Cycloalkyl ausgewählt ist, wobei C₃-C₁₀-Cycloalkyl wahlweise mit einer, zwei oder drei Gruppen ausgewählt aus C₁-C₃-Alkyl substituiert ist;
R₄ und R₅ unabhängig voneinander aus H, C₁-C₆-Alkyl, X und -C1-C6-Alkylen-X ausgewählt sind, wobei X ausgewählt ist aus
(i) C₃-C₆-Cycloalkyl;
(ii) Heteroaryl, das 5 bis 6 Ringatome enthält, wobei eines, zwei oder drei der Ringatome unabhängig voneinander aus N, NH, N(C₁-C₃-Alkyl), O und S ausgewählt sind;
(iii) Heterocyclyl, das drei bis sechs Ringatome enthält, wobei 1, 2 oder 3 der Ringatome unabhängig voneinander aus N, NH, N(C₁-C₃-Alkyl), O und S ausgewählt sind; und
(iv) Phenyl;
wobei C₃-C₆-Cycloalkyl, Heterocyclyl, Heteroaryl und Phenyl jeweils wahlweise mit einem, zwei oder drei Substituenten substituiert sind, die unabhängig voneinander aus Halogen, Cyano, C₁-C₆-Alkyl, Hydroxyl und C₁-C₆-Alkoxy ausgewählt sind;
oder R₄ und R₅ zusammen mit dem Stickstoff, an den sie gebunden sind, Heterocyclyl bilden, das 4 bis 6 Ringatome enthält; wobei das Heterocyclyl nicht mehr als zwei Ringheteroatome enthält, einschließlich des an R₄ und R₅ gebundenen Stickstoffatoms, und das zweite Ringheteroatom, wenn vorhanden, unabhängig aus N-C₁-C₆-Alkyl, O und S ausgewählt ist; und wobei das Heterocyclyl wahlweise mit einem, zwei oder drei Substituenten substituiert ist, die unabhängig voneinander aus Halogen, Cyano, C₁-C₆-Alkyl und C₁-C₆-Alkoxy ausgewählt sind;
R₆ aus -OH, C₁-C₆-Alkoxy, -OC(O)-C₁-C₆-Alkyl und -OC(O)Phenyl ausgewählt ist; und
R₇ H oder C₁-C₆-Alkyl ist.

2. Verbindung nach Anspruch 1, welche eine Verbindung der Formel IIa ist: 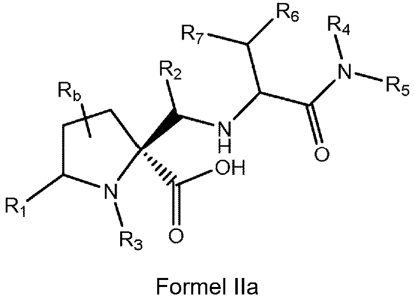 wobei R_{b}, R₁, R₂, R₃, R₄, R₅, R₆ und R₇ wie in Anspruch 1 definiert sind.

3. Verbindung nach Anspruch 2, welche eine Verbindung der Formel IIb ist: 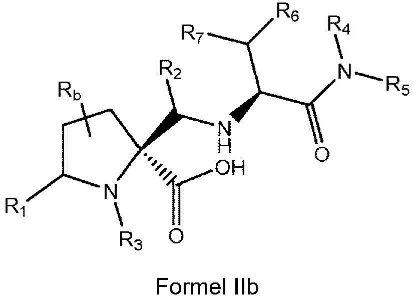 **wobei** R_{b}, R₁, R₂, R₃, R₄, R₅, R₆ und R₇ wie in Anspruch 1 definiert sind.

4. Verbindung nach Anspruch 3, welche eine Verbindung der Formel IIc ist: 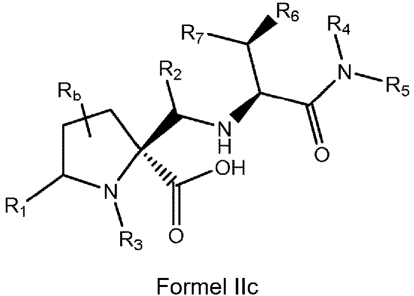 wobei R_{b}, R₁, R₂, R₃, R₄, R₅, R₆ und R₇ wie in Anspruch 1 definiert sind.

5. Verbindung nach Anspruch 1, wobei:
R₄ und R₅ unabhängig voneinander aus H und C₁-C₆-Alkyl ausgewählt sind; oder
R₄ und R₅ zusammen mit dem Stickstoff, an den sie gebunden sind, eine Heterocyclylgruppe bilden, die 4 bis 6 Ringatome enthält; wobei die Heterocyclylgruppe nicht mehr als zwei Ringheteroatome enthält, einschließlich des an R₄ und R₅ gebundenen Stickstoffatoms, und das zweite Ringheteroatom, wenn vorhanden, unabhängig aus N-C₁-C₆-Alkyl, O und S ausgewählt ist; und wobei die Heterocyclylgruppe wahlweise mit einem, zwei oder drei Substituenten substituiert ist, die unabhängig voneinander aus Halogen, Cyano, C₁-C₆-Alkyl und C₁-C₆-Alkoxy ausgewählt sind.

6. Verbindung nach Anspruch 5, wobei R₁, R₂, R₄ und R₅ jeweils H sind.

7. Verbindung nach Anspruch 1, welche aus den folgenden Verbindungen ausgewählt ist:

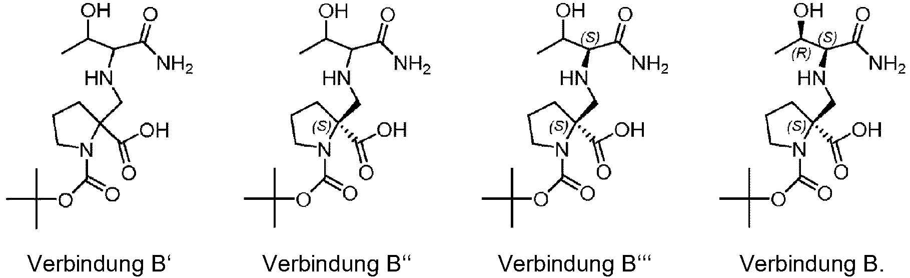

8. Verbindung nach Anspruch 7, welche Verbindung B ist.

9. Verbindung der Formel III: 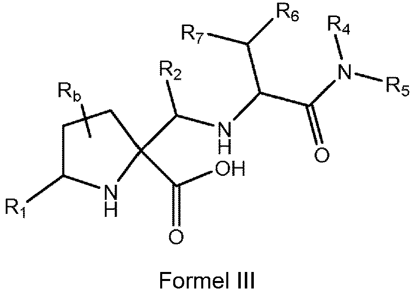 wobei:
R_{b} aus H, Halogen, Cyano und C₁-C₆-Alkyl ausgewählt ist;
R₁ H oder C₁-C₆-Alkyl ist;
R₂ H oder C₁-C₆-Alkyl ist;
R₄ und R₅ unabhängig voneinander aus H, C₁-C₆-Alkyl, X und -C₁-C₆-Alkylen-X ausgewählt sind, wobei X ausgewählt ist aus
(i) C₃-C₆-Cycloalkyl;
(ii) Heteroaryl, das 5 bis 6 Ringatome enthält, wobei eines, zwei oder drei der Ringatome unabhängig voneinander aus N, NH, N(C₁-C₃-Alkyl), O und S ausgewählt sind;
(iii) Heterocyclyl, das drei bis sechs Ringatome enthält, wobei 1, 2 oder 3 der Ringatome unabhängig voneinander aus N, NH, N(C₁-C₃-Alkyl), O und S ausgewählt sind; und
(iv) Phenyl;
wobei C₃-C₆-Cycloalkyl, Heterocyclyl, Heteroaryl und Phenyl jeweils wahlweise mit einem, zwei oder drei Substituenten substituiert sind, die unabhängig voneinander aus Halogen, Cyano, C₁-C₆-Alkyl, Hydroxyl und C₁-C₆-Alkoxy ausgewählt sind;
oder R₄ und R₅ zusammen mit dem Stickstoff, an den sie gebunden sind, ein Heterocyclyl bilden, das 4 bis 6 Ringatome enthält; wobei das Heterocyclyl nicht mehr als zwei Ringheteroatome enthält, einschließlich des an R₄ und R₅ gebundenen Stickstoffatoms, und das zweite Ringheteroatom, wenn vorhanden, unabhängig aus N-C₁-C₆-Alkyl, O und S ausgewählt ist; und wobei das Heterocyclyl wahlweise mit einem, zwei oder drei Substituenten substituiert ist, die unabhängig voneinander aus Halogen, Cyano, C₁-C₆-Alkyl und C₁-C₆-Alkoxy ausgewählt sind;
R₆ aus -OH, C₁-C₆-Alkoxy, -OC(O)-C₁-C₆-Alkyl und -OC(O)Phenyl ausgewählt ist; und
R₇ H oder C₁-C₆-Alkyl ist.

10. Verbindung nach Anspruch 9, welche eine Verbindung der Formel IIIa ist: 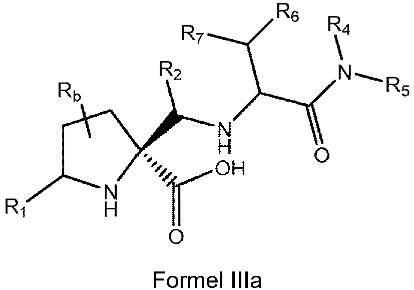 wobei R_{b}, R₁, R₂, R₄, R₅, R₆ und R₇ wie in Anspruch 9 definiert sind.

11. Verbindung nach Anspruch 10, welche eine Verbindung der Formel IIIb ist: 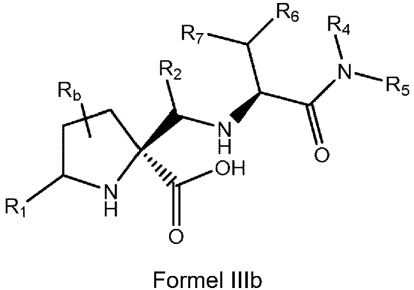 wobei Rₑ, R₁, R₂, R₄, R₅, R₆ und R₇ wie in Anspruch 9 definiert sind.

12. Verbindung nach Anspruch 11, welche eine Verbindung der Formel IIIc ist: 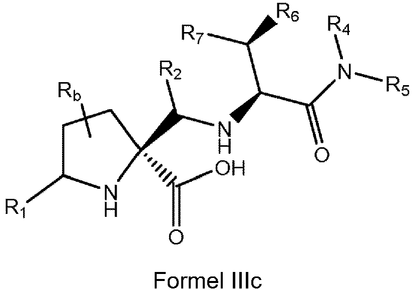 wobei R_{b}, R₁, R₂, R₄, R₅, R₆ und R₇ wie in Anspruch 9 definiert sind.

13. Verbindung nach Anspruch 9, wobei:
R₄ und R₅ unabhängig voneinander aus H und C₁-C₆-Alkyl ausgewählt sind; oder
R₄ und R₅ zusammen mit dem Stickstoff, an den sie gebunden sind, ein Heterocyclyl bilden, das 4 bis 6 Ringatome enthält; wobei das Heterocyclyl nicht mehr als zwei Ringheteroatome enthält, einschließlich des an R₄ und R₅ gebundenen Stickstoffatoms, und das zweite Ringheteroatom, wenn vorhanden, unabhängig aus N-C₁-C₆-Alkyl, O und S ausgewählt ist; und wobei das Heterocyclyl wahlweise mit einem, zwei oder drei Substituenten substituiert ist, die unabhängig voneinander aus Halogen, Cyano, C₁-C₆-Alkyl und C₁-C₆-Alkoxy ausgewählt sind.

14. Verbindung nach Anspruch 13, wobei R₁, R₂, R₄ und R₅ jeweils H sind.

15. Verbindung nach Anspruch 9, welche aus den folgenden Verbindungen ausgewählt ist:

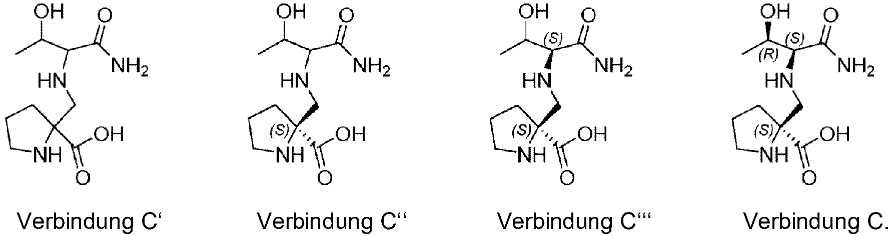

## Revendications

1. Composé de formule II : 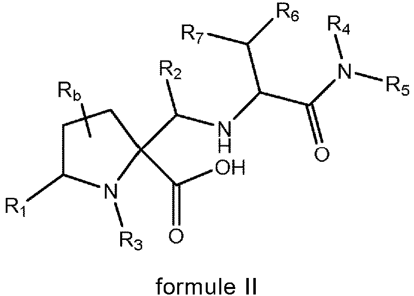 dans lequel :
R_{b} est sélectionné parmi H, halogène, cyano et alkyle en C₁ à C₆ ;
R₁ est H ou alkyle en C₁ à C₆ ;
R₂ est H ou alkyle en C₁ à C₆ ;
R₃ est sélectionné parmi un alkyle en C₁ à C₆ et C(O)OR₈ ;
R₈ est sélectionné parmi un alkyle en C₁ à C₆, haloalkyle en C₁ à C₆, alcényle en C₂ à C₆, alkynyle en C₂ à C₆, cycloalkyle en C₃ à C₁₀ et -CH₂-cycloalkyle en C₃ à C₁₀, dans lequel cycloalkyle en C₃ à C₁₀ est facultativement substitué par un, deux, ou trois groupes sélectionnés parmi un alkyle en C₁ à C₃ ;
R₄ et R₅ sont indépendamment sélectionnés parmi H, alkyle en C₁ à C₆, X et alkylène-X en C1 à C6, dans lequel X est sélectionné parmi
(i) un cycloalkyle en C₃ à C₆ ;
(ii) un hétéroaryle contenant 5 à 6 atomes de cycle dans lequel un, deux ou trois des atomes de cycle sont indépendamment sélectionnés parmi N, NH, N(alkyle en C₁ à C₃), O et S ;
(iii) hétérocyclyle contenant trois à six atomes de cycle dans lequel 1, 2 ou 3 des atomes de cycle sont indépendamment sélectionnés parmi N, NH, N(alkyle en C₁ à C₃), O et S ; et
(iv) un phényle ;
dans lequel cycloalkyle en C₃ à C₆, hétérocyclyle, hétéroaryle et phényle sont chacun facultativement substitués par un, deux ou trois substituants indépendamment sélectionnés parmi un halogène, cyano, alkyle en C₁ à C₆, hydroxyle, et alcoxy en C₄ à C₆ ;
ou R₄ et R₅ conjointement avec l'azote auquel ils sont attachés forment un hétérocyclyle contenant 4 à 6 atomes de cycle ; dans lequel l'hétérocyclyle ne contient pas plus de deux hétéroatomes de cycle incluant l'atome d'azote attaché à R₄ et R₅, et le deuxième hétéroatome de cycle, le cas échéant, est indépendamment sélectionné parmi un N-alkyle en C₁ à C₆, O et S ; et dans lequel l'hétérocyclyle est facultativement substitué par un, deux ou trois substituants indépendamment sélectionnés parmi un halogène, cyano, alkyle en C₁ à C₆ et alcoxy en C₁ à C₆ ;
R₆ est sélectionné parmi -OH, alcoxy en C₁ à C₆, -OC(O)-alkyle en C₁ à C₆ et -OC(O)phényle ; et
R₇ est H ou alkyle en C₁ à C₆.

2. Composé selon la revendication 1, qui est un composé de formule IIa : 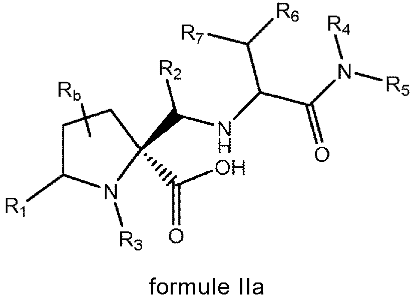 dans lequel R_{b}, R₁, R₂, R₃, R₄, R₅, R₆, et R₇ sont tels que définis dans la revendication 1.

3. Composé selon la revendication 2, qui est un composé de formule IIb : 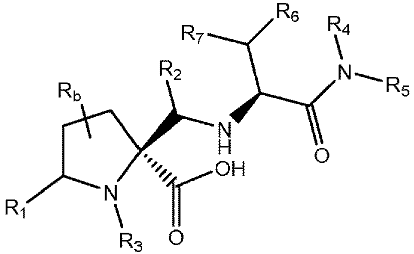 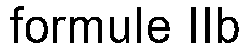 dans lequel R_{b}, R₁, R₂, R₃, R₄, R₅, R₆, et R₇ sont tels que définis dans la revendication 1.

4. Composé selon la revendication 3, qui est un composé de formule IIc : 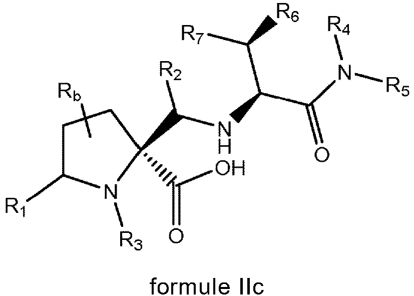 dans lequel R_{b}, R₁, R₂, R₃, R₄, R₅, R₆, et R₇ sont tels que définis dans la revendication 1.

5. Composé selon la revendication 1, dans lequel :
R₄ et R₅ sont indépendamment sélectionnés parmi H et alkyle en C₄ à C₆ ; ou
R₄ et R₅ conjointement avec l'azote auquel ils sont attachés forment un hétérocyclyle contenant 4 à 6 atomes de cycle ; dans lequel l'hétérocyclyle ne contient pas plus de deux hétéroatomes de cycle incluant l'atome d'azote attaché à R₄ et R₅, et le deuxième hétéroatome de cycle, le cas échéant, est indépendamment sélectionné parmi un N-alkyle en C₁ à C₆, O et S ; et dans lequel l'hétérocyclyle est facultativement substitué par un, deux ou trois substituants indépendamment sélectionnés parmi un halogène, cyano, alkyle en C₁ à C₆ et alcoxy en C₁ à C₆.

6. Composé selon la revendication 5, dans lequel chacun parmi R₁, R₂, R₄ et R₅ est H.

7. Composé selon la revendication 1, qui est sélectionné parmi les composés suivants :

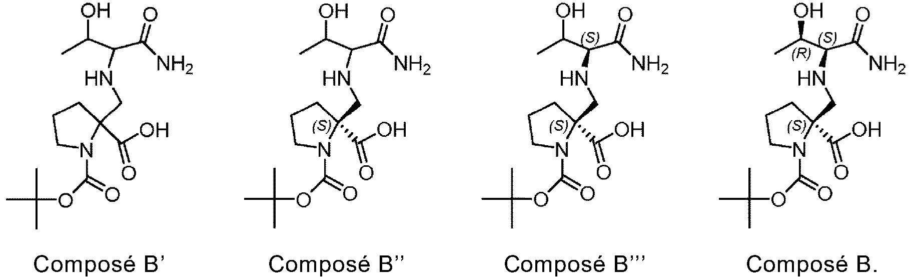

8. Composé selon la revendication 7, qui est le Composé B.

9. Composé de formule III : 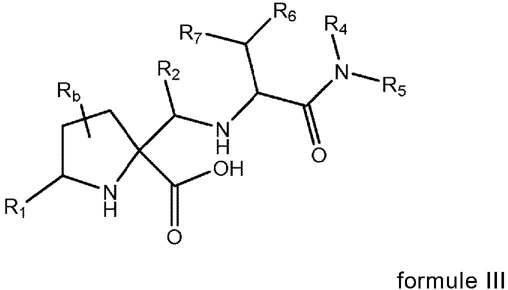 dans lequel :
R_{b} est sélectionné parmi H, halogène, cyano et alkyle en C₁ à C₆ ;
R₁ est H ou alkyle en C₁ à C₆ ;
R₂ est H ou alkyle en C₁ à C₆ ;
R₄ et R₅ sont indépendamment sélectionnés parmi H, alkyle en C₁ à C₆, X et alkylène-X en C₁ à C₆, dans lequel X est sélectionné parmi
(i) un cycloalkyle en C₃ à C₆ ;
(ii) hétéroaryle contenant 5 à 6 atomes de cycle dans lequel un, deux ou trois des atomes de cycle sont indépendamment sélectionnés parmi N, NH, N(alkyle en C₁ à C₃), O et S ;
(iii) hétérocyclyle contenant trois à six atomes de cycle dans lequel 1, 2 ou 3 des atomes de cycle sont indépendamment sélectionnés parmi N, NH, N(alkyle en C₁ à C₃), O et S ; et
(iv) un phényle ;
dans lequel un cycloalkyle en C₃ à C₆, hétérocyclyle, hétéroaryle et phényle sont chacun facultativement substitués par un, deux ou trois substituants indépendamment sélectionnés parmi un halogène, cyano, alkyle en C₁ à C₆, hydroxyle, et alcoxy en C₁ à C₆ ;
ou R₄ et R₅ conjointement avec l'azote auquel ils sont attachés forment un hétérocyclyle contenant 4 à 6 atomes de cycle ; dans lequel l'hétérocyclyle ne contient pas plus de deux hétéroatomes de cycle incluant l'atome d'azote attaché à R₄ et R₅, et le deuxième hétéroatome de cycle, le cas échéant, est indépendamment sélectionné parmi un N-alkyle en C₁ à C₆, O et S ; et dans lequel l'hétérocyclyle est facultativement substitué par un, deux ou trois substituants indépendamment sélectionnés parmi un halogène, cyano, alkyle en C₁ à C₆ et alcoxy en C₁ à C₆ ;
R₆ est sélectionné parmi -OH, alcoxy en C₁ à C₆, -OC(O)-alkyle en C₁ à C₆ et -OC(O)phényle ; et
R₇ est H ou alkyle en C₁ à C₆.

10. Composé selon la revendication 9, qui est un composé de formule IIIa : 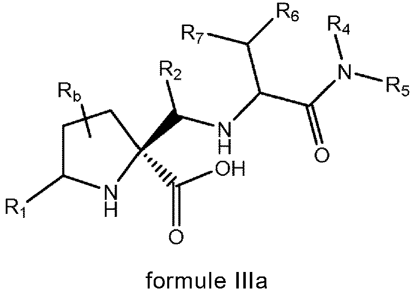 dans lequel R_{b}, R₁, R₂, R₄, R₅, R₆, et R₇ sont tels que définis dans la revendication 9.

11. Composé selon la revendication 10, qui est un composé de formule IIIb : 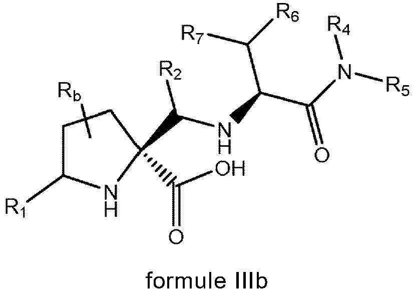 dans lequel R_{b}, R₁, R₂, R₄, R₅, R₆, R₇, sont tels que définis dans la revendication 9.

12. Composé selon la revendication 11, qui est un composé de formule IIIc : 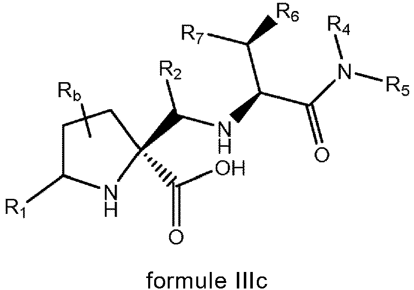 dans lequel R_{b}, R₁, R₂, R₄, R₅, R₆, R₇, sont tels que définis dans la revendication 9.

13. Composé selon la revendication 9, dans lequel :
R₄ et R₅ sont indépendamment sélectionnés parmi H et alkyle en C₁ à C₆ ; ou
R₄ et R₅ conjointement avec l'azote auquel ils sont attachés forment un hétérocyclyle contenant 4 à 6 atomes de cycle ; dans lequel l'hétérocyclyle ne contient pas plus de deux hétéroatomes de cycle incluant l'atome d'azote attaché à R₄ et R₅, et le deuxième hétéroatome de cycle, le cas échéant, est indépendamment sélectionné parmi un N-alkyle en C₁ à C₆, O et S ; et dans lequel l'hétérocyclyle est facultativement substitué par un, deux ou trois substituants indépendamment sélectionnés parmi un halogène, cyano, alkyle en C₁ à C₆ et alcoxy en C₁ à C₆.

14. Composé selon la revendication 13, dans lequel chacun parmi R₁, R₂, R₄ et R₅ est H.

15. Composé selon la revendication 9, qui est sélectionné parmi les composés suivants :
